(19) **Europäisches Patentamt / European Patent Office / Office européen des brevets**

(11) **EP 3 680 913 B1**

(12) **EUROPEAN PATENT SPECIFICATION**

(45) Date of publication and mention
of the grant of the patent:
**29.01.2025 Bulletin 2025/05**

(21) Application number: **19211641.6**

(22) Date of filing: **02.12.2013**

(51) International Patent Classification (IPC):
**G16H 10/20** *(2018.01)* **G16H 40/67** *(2018.01)*
**G16H 15/00** *(2018.01)* **G16H 50/30** *(2018.01)*
**G16H 20/60** *(2018.01)*

(52) Cooperative Patent Classification (CPC):
**G16H 10/20; G16H 15/00; G16H 20/60;**
**G16H 40/67; G16H 50/30**

(54) **AUTOMATED HEALTH DATA ACQUISITION, PROCESSING AND COMMUNICATION SYSTEM**

AUTOMATISIERTES SYSTEM ZUR ERFASSUNG, VERARBEITUNG UND ÜBERMITTLUNG VON GESUNDHEITSDATEN

SYSTÈME AUTOMATISÉ D'ACQUISITION, DE TRAITEMENT ET DE COMMUNICATION DE DONNÉES DE SANTÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2012 US 201261732203 P**

(43) Date of publication of application:
**15.07.2020 Bulletin 2020/29**

(62) Document number(s) of the earlier application(s) in accordance with Art. 76 EPC:
**13849993.4 / 2 926 285**

(73) Proprietor: **dacadoo ag**
**8008 Zurich (CH)**

(72) Inventors:
• **Leason, David**
 **Chappaqua, NY New York 10514 (US)**
• **Ohnemus, Peter**
 **8604 Herliberg (CH)**
• **Naef, Andre**
 **8057 Zurich (CH)**
• **Heuer, Manuel**
 **8702 Zollikon (CH)**

(74) Representative: **Williams Powell**
**44 Prospect Place**
**Bromley, Kent BR2 9HN (GB)**

(56) References cited:
**WO-A1-2012/050969    US-A1- 2009 113 016**
**US-A1- 2011 069 615    US-A1- 2012 042 004**
**US-B1- 6 751 657**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Cross-Reference to Related Applications**

The present application claims priority to U.S. Patent application No.: 61/732,203, filed November 30, 2012.

**Field**

The present application relate, generally, to networking and, more particularly, to a data acquisition, processing and communication system relating to an individual's health.

Background

Despite advances in many areas of technology, there are still barriers to assessing the relative health of a person in a rapid, cost effective, and timely manner. With the increase in health care costs and prevalence of diseases related to unhealthy lifestyles such as diabetes and heart disease, it is important to assess the relative health of individuals, and this has not been adequately addressed. In many areas of the world, access to doctors is limited. Even in the developed world, a doctor's time is considered a precious commodity and there are often long waiting lists and doctor-to-$pecialist referral systems have to be navigated before being seen. In more developed countries the ratio of doctors to the population can be on the order of 1:1,000 persons, while in less developed countries the ratio can be 1:100,000. There are also cost barriers to having access to a doctor because an appointment with a doctor can be very expensive, especially if an individual does not have any health insurance or lacks sufficient coverage. Accordingly, it can be very difficult to gain access to medical professionals in order to receive information about one's health.

Even individuals that have access to his or her health information, the mechanisms for conveying that information to others is lacking or non-existent. Privacy laws restrict the type of information that can be shared and the manner in which it can be shared. Privacy laws relating to health information are particularly strict in regard to the information that can be shared. This is to protect a person from disclosure of sensitive information. Accordingly, the sharing of health related information is generally discouraged. It is also difficult to share health related information with friends and family. Often health information is only verbally conveyed by a doctor to a patient, or the patient will only receive paper copies of lab test results . Systems are lacking for easily sharing such information with others, especially with large groups of persons located in geographically remote locations.

Furthermore, programs aimed at improving an individual's diet are usually based on an assessment of the type and the amount of food consumed using so called Food Frequency Questionnaires (FFQs). Based on the results, the programs give a "roadmap." For most users, this "roadmap" is relatively easy to follow and many of them achieve their nutritional goals. Unfortunately, many changes fail to become second nature to the user, and he or she often reverts back to 'old' behaviors. Another limitation of FFQs is that people tend to forget when and what they eat and often underestimate the amount and frequency of eating. Accurate documentation is also a laborious and time-consuming task, which often leads to loss of motivation.

The present application addresses these and other concerns.

WO 2012/050969 A1 discloses a user health score computation.

**Summary**

In accordance with one or more implementations, a system and method provide health-related information to a computing device. A user interface may be provided on a computing device, and assessment information associated with an assessment of a user's health can be received from the computing device via the user interface. Further, sensed information of the type that is associated with at least one of biological information, physiological information and physical activity of the user can be received from a different device which is configured to sense information. Moreover, a processing subsystem that includes a processor and processor readable media can be configured to process the sensed information, via, to provide processed user information, and to determine health-related information, via the processing subsystem, using the assessment information and the processed user information. Furthermore, the processed user information and the health-related information can be transmitted via a communication subsystem to the computing device, and the health-related information can be provided at the computing device via the user interface substantially contemporaneously with the reception of the sensed information.

In one or more implementations, determining the health-related information can comprise assigning at least one weighting factor associated with the assessment information, and calculating a masked composite numerical value using the at least one weighting factor and the processed user information in accordance with an algorithm to generate a health score. Furthermore, the system and method can include determining, via the processing subsystem, at least one of medical diagnostic information, medical benchmark information and medical analytic information, using the health-related information or the processed user information; and providing at least one of the medical diagnostic information, medical benchmark information and medical analytic information, at the computing device via the user interface.

In one or more implementations, medical information associated with at least one of the user's family medical history, the user's demographics and the user's metabolism, can be received and, wherein determining the health-related information further includes using the medical information. Moreover, the system and method can include receiving, from the different device which is configured to sense information, additional sensed information of the type that is associated at least one of biological information, physiological information and/or physical activity of the user; processing the additional sensed information, via the processing subsystem, to provide updated processed user information; determining updated health-related information, via the processing subsystem, using the updated processed user information; transmitting, via the communication subsystem, the updated processed user information and the updated health-related information to the computing device, and providing the updated health-related information at the computing device via the user interface. The system and method can include comparing, via the processing subsystem, at least two of the health-related information, the processed user information, the updated health-related information and the updated processed user information; determining feedback, via the processing subsystem, based on the comparison; and providing the feedback at the computing device via the user interface. The feedback can include at least one of an alert and a notification.

Moreover, in one or more implementations, the system and method can include receiving other user health-related information associated with at least one other user: comparing, via the processing subsystem, the health-related information with the other user health-related information to generate a comparison; and providing comparative information associated with the comparison at the computing device via the user interface. The comparative information can regard at least one of social relations, personal progress, reminders for input and private messaging. Moreover, the user interface can include a selectable option for regulating at least one of an amount of health-sedated information to be displayed, a type of health-related to be displayed, and a frequency of displaying information.

Preferred embodiments of the present application seek to provide a system and method that provide an assessment of the relative health of an individual that can be used as the basis of a fair comparison to other individuals having different ages, sex, medical status or lifestyles. The invention is defined by the appended claims.

Various features, aspects and advantages of the invention can be appreciated from the following detailed description and the accompanying drawing figures.

**Brief Description of the Drawing Figures**

Fig. 1 a diagram of an example hardware arrangement in accordance with an example implementation;

Fig. 2 illustrates functional elements of an example information processor

and/or workstation in accordance with an implementation of the present application;

Fig. 3A is a block diagram that illustrates functional building blocks associated with an implementation of the present application;

Fig. 3B is a schematic block diagram according to one or more embodiments of the present application;

Fig. 4A illustrates an example flowchart of steps associated with nutrition tracking in accordance with an implementation of the present application;

Figs. 4B and 4C illustrate example data entry display screens and controls in accordance with an implementation of the present application;

Fig. 4D illustrates examples of automatic data entry/import controls in accordance with an implementation of the present application;

Figs. 5 illustrates a list of activities provided via a mobile computing device in connection with device integration accordance with an implementation of the present application:

Fig. 6 illustrates steps associated with integrating a device in connection with server-side integration in accordance with one or more implementations of the present application;

Fig. 7 illustrates the interrelationship between variables associated with a person in the calculation of a health score, in accordance with an implementation of the present application:

Fig. 8 illustrates an example "body report" divided in accordance with an implementation of the present application;

Fig. 9 illustrates an example calendar view for users to review their fitness activities and receive feedback in accordance with an implementation of the present application;

Fig. 10A illustrates a graphical indication of a user's goal activities, including in terms of energy and duration in accordance with an implementation of the present application;

Fig. 10B illustrates an example display screen associated with current goals and reached goals for an individual;

Fig. 10C illustrates an example interface for defining and achieving goals, in accordance with the present application;

Fig. 10D illustrates an example display screen enabling creation of goals as a function of achievements, workouts and weight;

Figs. 11A and 11B illustrate example screen displays associated with achievements and the progress of users;

Fig. 12 illustrates an example display screen associated with a public challenge, in accordance with an implementation of the present application;

Fig. 13 illustrates an example display screen associated with news and notifications, in accordance with an implementation of the present application;

Figs. 14 illustrates an avatar, "Q," in accordance with implementations of the present application;

Fig. 15 illustrates an implementation of the present application that separates a link between health information and account information; and

Fig. 16 illustrates mobile computing devices running mobile applications, in accordance with implementations of the present application.

Detailed Description

In one or more example implementations, the present application provides a computer-implemented system and method configured to acquire health-related and/or medical-related data, and to process the data, for example, for diagnostic, benchmarking, analytic and/or data distribution (e.g., reporting) purposes. For example, the systems and methods herein provide for providing feedback substantially in real-time via an on-line and/or mobile platform. Using the systems and methods disclosed herein, information can be received from user devices, and the information can be processed to provide various form of feedback, such as alerts and notifications. Related information can be, thereafter, received, processed, thereby providing additional feedback (e.g., a form of a feedback loop).

In one or more implementations, one or more rule engines can be provided that periodically and/or continuously process information and that generate notifications to users. Implementations can depend on a respective subsystem (e.g., data gathering subsystems, data communication subsystems, data processing subsystems) and one or more corresponding notification features. Moreover, one or more notification generating rule engines can be part of individual subsystems generating those notifications. The notification features can include core information elements that are useful for the feedback process. Generally, notifications can include questionnaires or prompts for information, and can be presented by an interactive interface, such as an avatar. The result can include an infrastructure configured for scheduling, processing, and delivering notifications over various channels.

In accordance with one or more implementations, a respective notification type can be assigned to a domain. Moreover, users can choose a respective "channel" used for receiving notifications based on the respective domain of a notification. For example, the following notification domains can be supported; Social; Personal Progress; Requests/Reminders for Input; and Private Messaging. Further, a default set of one or more channels can be assigned to each domain, which can be overridden by users. For example, the following channels can be supported: Internet web site; mobile device software application ("mobile app"); e-mail; SMS; and mobile device push. Notifications can also be exported to a partner system, such as a customer relations management ("CRM") system, for further processing. On the web and/or in a mobile app, a user interface can include a suitable inbox for users to review notifications easily and quickly. The user interface can distinguish between notifications that can be new and previously reviewed.

Notifications generated by one or more rule engines can be assigned a priority between zero and one, which priority can be static or be calculated dynamically, based on the specific content/parameters of the notification. In order to prevent overwhelming users with excessive amounts of information, notifications can be provided in accordance with various system parameters. For example, a cap specifying a maximum number of notifications of a particular type that is delivered per interval can be employed. If the cap is surpassed, then only those notifications assigned a high priority may be pushed to the user. Users can partially in fluence the cap by selecting an option for being provided information, such as "show me more Jess of this" functionality in a user interface ("UI"), In addition, a cool-down value can be employed that specifies a minimum time that should pass between notifications of a specific type or that meet specific content/parameters. Moreover, a folding function can be used that allows for combining multiple notifications into one (e.g, folding three friend suggestions into a single notification).

In one or more implementations, sensed information of the type that is associated with at least one of biological information, physiological information and physical activity of the user can be received from one or more devices is configured to sense information. In addition to displaying or otherwise providing information representing the user's activity, as well as biological and/or physiological information associated with the user, the present application can be configured to provide reminders and/or notifications that are associated with adherence to medication, behavior (e.g., activity or abstaining from certain activity), or for monitoring one or more medical conditions. In addition to displaying information, a vibration mechanism (as known in the art) or other suitable configuration can be provided to provide an alert to a user. The user's cell phone, for example, can vibrate to alert the user, for example, to take medication (e.g., a beta blocker, diabetes II medicine, blood pressure medication, or the like). Alternatively, the alert may remind the user to take some action, such as to draw blood to check blood glucose levels, to check heart rate or blood pressure, or to take some other action, such as to exercise (e.g., take a walk or participate in a challenge), or to consume food (or stop consuming food).

in one or more implementations, information, such as health-related information, alerts, notifications or the like, can be provided can be provided at the computing device via a user interface substantially contemporaneously with the reception of the sensed information.

In addition, the present application can be configured to provide audio information, In one or more implementations, one or more speakers and audio components can provide audio-based information. In addition, a microphone can be provided to receive voice commands and/or audio input. Moreover, a camera can capture still and/or moving images. The ability to send and receive multimedia content (e.g., audio and/or visual content) provides additional functionality associated with, for example, the user to interact with others in various ways.

In addition, information can be processed and associated with exercise and other workouts. Information, such as kilometer times, significant changes in heart rate, or guided training information, such as interval trainings, can be provided to a user substantially in real-time. Information may be displayed, and/or provided as multimedia-content,

In one or more implementations, the present application provides a notification scheduler that accepts notifications for delivery to users via particular channels. Once a notification has been submitted to the scheduler service, the notification can be placed in a queue, and one or more processors can then operate on the notifications queue(s). For example, each processor, while running on a queue, can take actions such as dropping, delivering, keeping or folding notifications. This provides for significant flexibility is provided, For example, it is possible to keep each notification queued for a particular (or arbitrary) amount of time. Even holding a notification for a very short amount of time can significantly increase the chance of folding a notification with another that is generated only a small fraction of time later.

In operation, an initial testing process may be provided in which questions and/or information is presented, and users can be offered an opportunity to answer various questions, such as to determine whether the content understandable/clear, meaningful, relevant, fun and/or entertaining. Additionally, free-text responses may be provided via voice-input, text-input controls (e.g., text boxes), or other graphical screen elements. Responses to questions can be answered via a graphical slider control that provides options, such as "not at all" to "very much," which can correspond to numerical values, such as 0.0 to 1.0. This information can be stored together with the date of submission and user identifier, such as an anonymous user ID. In one or more implementations, repeated submissions that are received by the same user (or user device) relating to the same topic can overwrite the previous submission. Further, a simple text report showing the mean value and standard deviation of the answers, followed by one or more received (i.e., non-empty) comments can be provided. Moreover, a notification catalogue can be provided that can be operable as a function of declarative logic, and with relatively little specific programming to implement the one or more rules engines.

By way of overview and introduction, the present application is described in detail in connection with a distributed system in which data acquisition, data storage, and data processing can be used to produce a numerical score as a basis for assessing the relative health of a user. In an implementation, a computer-based application is provided for the collection of health-related parameters of a user and a user interface for the presentation (e.g., display) of data. The computer-based application can be implemented via a microcontroller that includes a processor, a memory and code executing therein so as to configure the processor to perform at least some of the functionality described herein. The memory can store data and instructions suitable for controlling the operation of one or more processors. An implementation of memory can include, by way of example and not limitation, a random access memory (RAM), a hard drive, or a read only memory (ROM). One of the components stored in the memory is a program. The program includes instructions that cause the processor to execute steps that implement the methods described herein. The program can be implemented as a single module or as a plurality of modules that operate in cooperation with one another. The program can include software that can be used in connection with one or more implementations of the present application.

A communication subsystem can be provided for communicating information from the microprocessor to the user interface, such as an external device (e.g., handheld unit or a computer that is connected over a network to the communication subsystem). Information can be communicated by the communication subsystem in a variety of ways including Bluetooth, Wi-Fi, Wi-Max, RF transmission, near-field communications or other suitable communication protocol. A number of different network topologies can be utilized in a conventional manner, such as wired, optical, 3G, 4G or other suitable networking protocol,

The communication subsystem can be part of a communicative electronic device including, by way of example, a smart phone or cellular telephone, a personal digital assistant (PDA), tablet computer, netbook, laptop computer, or other computing device. For instance, the communication subsystem can be directly connected through a device such as a smartphone such as an iPhone, Google Android Phone, BlackBerry, and Microsoft Windows Mobile enabled phone, or a device such as a heart rate or blood pressure monitor, weight measurement scales, exercise equipment or the like. One or more of these devices can include or otherwise interface with a module or communication unit with the subsystem to allow information and control signals to flow between the subsystem and the external user interface device. The communication sub-system can cooperate with a conventional communicative device, or can be part of a device that is dedicated to the purpose of communicating information processed by the microcontroller.

When a communicative electronic device such as the types noted above can be used as an external user interface device, the display, processor, and memory of such devices can be used to process the health related information in order to provide a numerical assessment. Otherwise, the system can include a display and a memory that are associated with the external device and used to support data communication in real-time or otherwise. More generally, the system includes a user interface, which can be implemented, in part, by software modules executing in the processor of the microcontroller or under control of the external device, In part, the user interface can also include an output device such as a display (e.g., the display). Display may include, for example, organic light emitting diode (OLED) displays, thin film transistor liquid crystal displays and plasma displays

In one or more implementations, biosensors can be used to collect and transmit health information about a user to one or more computing devices. The biosensor can be placed in contact with or within a user's body to measure vital signs or other health-related information of the user. For example, the biosensor can be a pulse meter that can be worn by the user in contact with the user's body so that the pulse of the user can be sensed, a heart rate monitor, an electrocardiogram device, a pedometer, a blood glucose monitor or other suitable device or system. A biosensor in accordance with the present application can include a communication module (e.g., a communication subsystem) so that the biosensor can transmit sensed data, either wired or wirelessly . The biosensor can communicate the sensed data to the user interface device, which in turn communicates that information to the microcontroller. Optionally, the biosensor can directly communicate the sensed the data to the microprocessor. The use of biosensors provides a degree of reliability by eliminating user error associated with manually entered and/or self-reported data,

Alternatively or in addition, the user can self-report his or her health related information by manually inputting the data. Thus, in an implementation, health-related data of a person can be entered manually into a computing device, and thereafter transmitted across a network to another device, such as a server computer.

The present application is configured to assign a numerical value that represents the relative health of an individual. Referred to herein, generally, as a "health score," the value can be used to assess to the individual's health based on health related information collected from a user. The health score is calculated based on the collected health information using an algorithm. The user or a communication subsystem provides the health-related information, for example in connection with one or more health parameters. Predetermined weighting factors are used to assign a relative value of each of the parameters that are used to calculate the health score. The user's health score is then calculated by combining the weighted parameters in accordance with an algorithm. By providing the health score, a user gets health-related feedback information and can make modifications in his/her lifestyles that can directly impact the user's health score and improve the user's health, more generally.

In one or more implementations, the present application calculates an "Effective Age" value, which represents an age that can be associated with the user based upon biometric and other information attributed to calculating the user's health score, notwithstanding the user's actual age. As a user modifies his or her lifestyle, which impacts the user's health score, the user can see his or her effective age changes.

In accordance with the present application, three interrelated components are included in calculating the user's health score: a metric health model ("MHM"), which includes subjective information from the user about who the user is; a quality of life model ("QLM"), which includes subjective information from the user about how the user feels; and a lifestyle model ("LSM") which includes subjective information from the user about the user lives. One or more weighting factors can be applied to each of these components. These components can be represented as percentage values. For example for MHM the weighting factor can be 35%, for QLM the weighting factor can be 20%, and for LSM the weighting factor can be 45%. The percentages can be static values, or can be dynamic. Example categories of input information that contribute to the values can include demographic information and anthropomorphic information (e.g., age, ethnicity, gender, height, weight, body-mass index and waist circumference), familial information (such as family histories, e.g., premature CVD, Diabetes, angina, heart attack hypertension), metabolic information (e.g., total serum cholesterol, high-density lipoprotein tsc/hdl, low-density lipoprotein, triglycerides, fasting blood glucose, systolic blood pressure, diastolic blood pressure, C-reactive protein, resting heart rate, and percent body fat), lifestyle-derived information (e.g., daily smoking and alcohol intake), pre-existing conditions (e.g., left ventricular hypertrophy, Type II Diabetes mellitus, hypertension, arrhythmia, Chronic Kidney Disease, MI, stroke, TIA, or Congestive Heart Failure) and self-assessment information. If fat is given in the input for MHM, the BMI may be generated by an internal function fat2bmi() with the BMI in the input, and it will take the smaller of the two.

In addition to calculating health score using estimates of cardiovascular and other risks associated with measurable parameters, such as blood pressure, weight, lipid levels or the like, the present application applies information associated with the MHM, QLM and LSM to further determine and/or estimate risks. For example, risks associated with the most common vascular and other biological elements can be derived from the results of information from studies that have been suitably, and which can be modified to provide consistency. A score associated with the MHM, for example, can include three factors that cover a very broad set of disease end-points and associated risk factors: a) direct vascular risks, which estimate the risks associated with major vascular events, such as stroke, or myocardial infarction; b) predecessor risks, which estimate the risks associated with major vascular risk factors, such as Type 2 diabetes or hypertension; and c) modulator risks, which scale the overall risk using risk factors not included in the other two components, such as alcohol consumption or certain aspects of nutrition. These modulator factors include parameters from both the QLM and LSM. Each of these components can include several models that can be combined to produce a single estimate of a health-risk event. The overall risk can be transformed into a score between $\Omega$ and 1,000, with 1,000 signifying perfect, but unattainable health.

In one or more implementations, a process of verifying data integrity in multiple stages can be provided. For example, input data structures include metadata that is processed and used in the calculation of the health score, The metadata can include various attributes in a first stage of the verification process, such as: required data, minimum value(s), maximum value(s), and default value(s). Data can be first checked for completeness, and values for missing data fields that passed the first stage can be imputed using one or more models, for example, based on the use of required fields only.

In connection with the Quality of Life model, a warning can be provided to a user after the first quality of life questionnaire is completed. In the event that a value is received that is above the 96th percentile of original survey data, the user can be provided with a message, such as a warning, that his or her responses appear to be unrealistic, and inviting the user to repeat the process to generate a new score. The message can include a statement that the benefit of the score would be lost if it is not taken seriously. In an embodiment, subsequent updates of a questionnaire are not checked for a determination of realistic values.

In accordance with the present application, one or more components are factored into a measurement to determine an extent to which lifestyle characteristics can impact a user's future health. Such components include fitness, nutrition, background physical activity, stress reduction, weight management, and smoking cessation. Two or more of these components can interrelate, which can be reflected in an associated individual and overall health scores. The weights with which the components contribute to an overall lifestyle score can be determined dynamically from two factors: (1) the sensitivity of the MHM score to changes in a set of modifiable risk factors (MRF) for a given user, and (2), a sensitivity matrix that relates the effect of each lifestyle component on each of the MRF. This mechanism leads to a recommendation to the user, based on a ranking in accordance with relevance of the factors that relate to the user's changing lifestyle. Further, the weights associated with each lifestyle component that contributes to the health score can be modified, with the most relevant factor receiving the highest weight. In one or more implementations, the priority of lifestyle components is provided to the user in a simple and visually compelling manner.

Also and in accordance with one or more implementations, the complete (or partial) health score can be validated in a prospective study. In such case, a collaboration of a sufficiently large cohort of users is used for those who regularly and periodically provide accurate data, and for whom health outcomes over time are available.

In one or more implementations, the LHM represents health-improvement efforts taken by a user and corresponding health-related consequences thereof. A percentage value can be attributed to the LHM component can be higher than, for example, the MHM or QLM components. Moreover, in an embodiment, various categories can be employed to monitor and quantify lifestyle characteristics that are strongly correlated with overall health. The categories can include fitness, nutrition, stress, background physical activity, weight-management and smoking cessation. These can be quantified, for example, using a double-buffer method, including a score component, a bonus component and a decay function, which can vary in value depending upon a particular lifestyle component.

Generally, each of the lifestyle components generates a score, such as in a range of 0-1,000. The scores can be combined using a dynamic weighing scheme based on the relevance of each for a given user and at a given time. The weights can be proportional to the relevance to the user at any given time. A discussion regarding an example weighing scheme in accordance with one or more implementations is provided below.

In an embodiment, a plurality of components is factored in a calculation of the MHM. For example, precursor risks are considered, in which a number of risk factors are used to determine a probability of developing a disease, such as a cardiovascular and/or cerebrovascular disease and certain cancers. Such probability may be estimated using a set of models derived from studies, which can be modified for consistency. The time horizon for these risks can be defined, for example, at four years, and the derived probabilities can be used in place of binary risk factors that can be used in the core risk models. In one or more implementations, the diseases and syndromes included as precursors are: chronic kidney disease; diabetes mellitus type II; hypertension; Metabolic Syndrome; and peripheral arterial disease.

In addition, several risk factors may be derived from lifestyle and metabolic characteristics. These risk factors may be not directly included in the core risk models that are quantified using models and data from studies, and can be used either as overall risk multipliers for an appropriate core risk model, or as remnant risks, such as in the case of smoking cessation. Examples of risks and factors as risk modulators can include: alcohol consumption; physical activity; nutrition; resting heart rate; heart rate recovery; smoking cessation; chronic stress; and depression. The input data for these models can include several sources, including inputs associated with family, demographics and metabolism, as well as other user inputs and parameters derived by internal models that use the inputs, data derived from the Quality of Life model, and data collected from one or more processes, substantially as shown and described herein.

In one or more implementations, a Metric health score includes a plurality of central estimators, which can be derived from data and one or more models, such as from one or more studies. The models can be modified and/or updated to provide an accurate Metric health score. Moreover, the models can be rescaled to produce approximate event probabilities for a fixed time horizon of time, such as for 10 years.

Examples of diseases and end-points included various calculations can be general cardiovascular disease; coronary heart disease; congestive heart, failure; myocardial infarction and stroke. In one or more particular cases, particular studies can include severity modifiers, such as death.

In connection with core risk models, weights and combinations thereof can be employed. For one or more diseases or end-points, several models can be included, which can result in given condition(s) that are combined using, for example, conservative probabilistic logic, and that can be internally weighted by the relative severity of the respective end-point under consideration. These individual estimates of risk can then themselves be weighted by relative severity and combined into an overall event probability, from which a score, such as ranging from 0 - 1,000 can derive a series of transformations. The parameters of these transformations also can be derived using data from known sources, such as the National Health and Nutrition Examination Survey (NHANES). Further, the Metric Score can be equalized to account for gender and age.

In accordance with the present application a recommendation or "focus engine" can be provided that informs users of one or more lifestyle components that the users should focus on to increase their health score efficiently. Users are provided with a prescription to focus on specific lifestyle issues to improve long-term health. The engine can do this by first calculating a user's room for improvement in the modifiable risk factors ("MRF"), Example modifiable risk factors can include, for example, weight, body-mass index, waist circumference, total serum cholesterol, high-density lipoprotein, low-density lipoprotein, triglycerides, tasting blood glucose, systolic blood pressure, diastolic blood pressure, C-reactive protein, resting heart rate, heart rate recovery, percent body fat, and smoking status. A calculation can be made regarding the difference in health score between a user's current value, and the value that would result if the user's MRF were ideal, e.g., at best values. It is recognized herein that a user may find that thinking in terms of MRF can be too abstract. For this reason, in a next step, the engine can calculate the combined weight of the MRFs for each lifestyle component. Lifestyle components, such as nutrition or fitness, are things that users may be more willing or able to relate to. Thus, presenting those lifestyle components ordered by the calculated weight gives a clear guidance to users as to which lifestyle components have the strongest effect on their overall health score and thus on their wellbeing.

The effect of changing any particular MRF from a current value to an ideal, best value can be quantified by determining the difference between the corresponding two metric health scores, thus producing a first recommendation, namely to focus on the MRF that produces the largest effect. In case this is construed to be overly abstract and/or unusable, a recommendation can be expressed in terms of lifestyle changes that most efficiently address the specific MRF. This results in a recommendation that is more usable and understandable for the user. For each of the MRF, $M_k$, there is an effect, $E_k = MHM(\{M_k\}) - MHM(\{M_k\{I_k\})$, where $I_k$ is the ideal value for the $k^{th}$ MRF.

To convert MRF modification to lifestyle change, a static matrix, referred to herein, generally, as a sensitivity matrix can be used. In accordance with this matrix, the columns represent the current lifestyle components, and the rows represent the MRF. The values can be a ranking of the lifestyle components by their effect on each of the MRF.

A discussion regarding respective component weights is now provided. In case $S_{um}$ is the rank (normalized to [0,1]) of the effect of the $n^{th}$ lifestyle factor on the $m^{th}$ MRF, one can define weights $w_n$ for each of the lifestyle factors as follows:

$$w_n = \frac{\tilde{w}_n}{\sum_{m \neq n} \tilde{w}_n}$$

where

$$\tilde{w}_n = \sum_m S_{nm} E_m$$

The engine can return $w_n$ as defined above to the platform, which can be used as relative weights for one or more of the lifestyle scores, The individual weighted scores, when summed and linearly normalized into the 0 - 1,1000 interval, define the overall Lifestyle Score, and 45% of the overall health score.

In addition to a focus engine, in one or more implementations the present application can include a recommendation normalization and engine. This can employ two lifestyle components; a fitness component; and a smoking cessation component (which can be active for current and previous smokers). Ranking is supported, and can use one or more other components, leading to a simple focus list. For example, a recommendation may be made that states, "the best immediate approach to increase a health score is to concentrate on fitness activities and improve your nutrition." This can be used even if there is no active nutrition tracker. In one or more implementations, to compute the Lifestyle Score, the platform can first renormalize the score to include only those components and trackers that are activated by the user.

As will become clear in accordance with the teachings herein, a sedentary lifestyle in most societies has dramatically increased the proportion of people who are overweight, have diabetes or suffer from heart failure, pressuring further the already stressed healthcare budgets of most developed countries. Insufficient activity has nearly had the same effect on life expectancy as smoking,

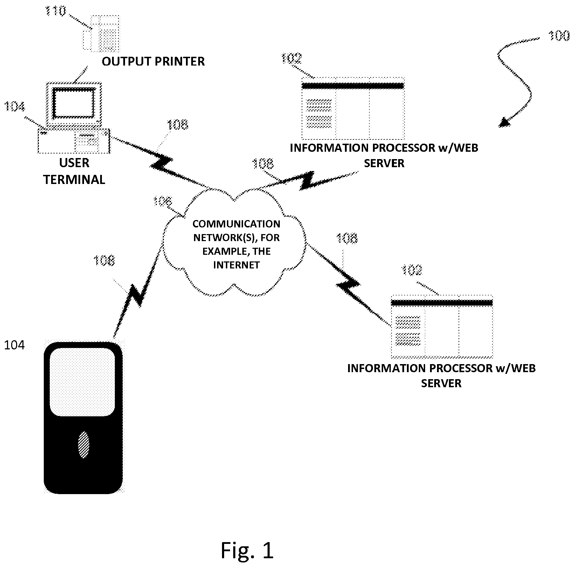

Referring now to the drawings figures in which like reference numerals refer to like elements, there is shown in Fig. 1 a diagram of an example hardware arrangement that operates for providing the systems and methods disclosed herein, and designated generally as health platform 100. Health platform 100 is preferably comprised of one or more information processors 102 coupled to one or more user computing devices 104 across communication network 106. User computing devices may include, for example, mobile computing devices such as tablet computing devices, smartphones, personal digital assistants or the like. Further, a plurality of sensing devices are included that transmit various health-related information to computing devices.

Information processor 102 preferably includes all necessary databases for the present application, including image files, metadata and other information relating to artwork, artists, and galleries. However, it is contemplated that information processor 102 can access any required databases via communication network 106 or any other communication network to whitch information processor 102 has access. Information processor 102 cam communicate devices comprising databases using any known communication method, including a direct serial, parallel, USB interface, or via a local or wide area network.

User computing devices 104 communicate with information processors 102 using data connections 108, which are respectively coupled to communication network 106. Communication network 106 can be any communication network, but is typically the Internet or some other global computer network. Data connections 108 can be any known arrangement for accessing communication network 106, such as dial-up serial line interface protocol/point-to-point protocol (SLIPP/PPP), integrated services digital network (ISDN), dedicated leased-line service, broadband (cable) access, frame relay, digital subscriber line (DSL), asynchronous transfer mode (ATM) or other access techniques.

User computing devices 104 preferably have the ability to send and receive data across communication network 106, and are equipped with web browsers to display the received data on display devices incorporated therewith. By way of example, user computing device 104 may be personal computers such as Intel Pentium-class computers, but are not limited to such computers. Other workstations which can communicate over a global computer network such as smartphones, tablet computers, personal digital assistants (PDAs) and mass-marketed Internet access devices such as WebTV can be used. In addition, the hardware arrangement of the present application is not limited to devices that are physically wired to communication network 106. Of course, one skilled in the art will recognize that wireless devices can communicate with information processors 102 using wireless data communication connections (e.g., Wi-Fi, ANT+, Bluetooth Low Energy ("BLE") or ZigBee).

In one or more implementations, the device in accordance with the present application may be configured to include a head-worn display that is configured to send, receive and display information as shown and described herein. For example, the present application may be configured with or in GOOGLE GLASS.

According to an embodiment of the present application, user computing device 104 provides user access to information processor 102 for the purpose of receiving and providing art-related information. The specific functionality provided by health platform 100, and in particular information processors 102, is described in detail below.

Health platform 100 preferably includes software that provides functionality described in greater detail herein, and preferably resides on one or more information processors 102 and/or user computing devices 104. One of the functions performed by information processor 102 is that of operating as a web server and/or a web site host. Information processors 102 typically communicate with communication network 106 across a permanent i.e. unswitched data connection 108. Permanent connectivity ensures that access to information processors 102 is always available.

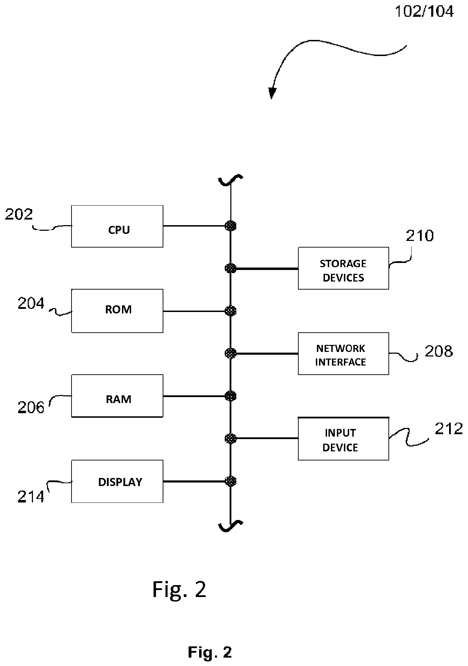

As shown in Fig. 2 the functional elements of each information processor 102 or workstation 104, and preferably include one or more central processing units (CPU) 202 used to execute software code in order to control the operation of information processor 102, read only memory (ROM) 204, random access memory (RAM) 206, one or more network interfaces 208 to transmit and receive data to and from other computing devices across a communication network, storage devices 210 such as a hard disk drive, flash memory, CD-ROM or DVD drive for storing program code, databases and application code, one or more input devices 212 such as a keyboard, mouse, track ball and the like, and a display 214.

The various components of information processor 102 need not be physically contained within the same chassis or even located in a single location. For example, as explained above with respect to databases which can reside on storage device 210, storage device 210 may be located at a site which is remote from the remaining elements of information processors 102, and may even be connected to CPU 202 across communication network 106 via network interface 208.

The functional elements shown in Fig. 2 (designated by reference numbers 202-214) are preferably the same categories of functional elements preferably present in user computing device 104. However, not all elements need be present, for example, storage devices in the case of PDAs, and the capacities of the various elements are arranged to accommodate expected user demand. For example, CPU 202 in user computing device 104 may be of a smaller capacity than CPU 202 as present in information processor 102. Similarly, it is likely that information processor 102 will include storage devices 210 of a much higher capacity than storage devices 210 present in workstation 104, Of course, one of ordinary skill in the art will understand that the capacities of the functional elements can be adjusted as needed.

The nature of the present application is such that one skilled, in the art of writing computer executed code (software) can implement the described functions using one or more or a combination of a popular computer programming language including but not limited to C++, VISUAL BASIC, JAVA, ACTIVEX, HTML 5, XML, ASP, SOAP, OBJECTIVE C, and C# and various web application development environments.

As used herein, references to displaying data on user computing device 104 refer to the process of communicating data to the workstation across communication network 106 and processing the data such that the data can be viewed on the user computing device 104 display 214 using a web browser or the like. The display screens on user computing device 104 present areas within control allocation health platform 100 such that a user can proceed from area to area within the control allocation health platform 100 by selecting a desired link. Therefore, each user's experience with control allocation health platform 100 will be based on the order with which (s)he progresses through the display screens. In other words, because the system is not completely hierarchical in its arrangement of display screens, users can proceed from area to area without the need to "backtrack" through a series of display screens. For that reason and unless stated otherwise, the following discussion is not intended to represent any sequential operation steps, but rather the discussion of the components of control allocation health platform 100.

Although the present application is described by way of example herein in terms of a web-based system using web browsers and a web site server (information processor 102), and with mobile computing devices (104) health platform 100 is not limited to that particular configuration. It is contemplated that control allocation health platform 100 can be arranged such that user computing device 104 can communicate with, and display data received from, information processor 102 using any known communication and display method, for example, using a non-Internet browser Windows viewer coupled with a local area network protocol such as the Internetwork Packet Exchange (IPX). It is further contemplated that any suitable operating system can be used on user computing device 104, for example, WINDOWS 3.X, WINDOWS 95, WINDOWS 98, WINDOWS 2000, WINDOWS CE, WINDOWS NT, WINDOWS XP, WINDOWS VISTA, WINDOWS 2000, WINDOWS XP, WINDOWS 7, WINDOWS 8, MAC OS, LINUX, IOS, ANDROID, WINDOWS PHONE 7, WINDOWS PHONE 8, and any suitable PDA or palm computer operating system.

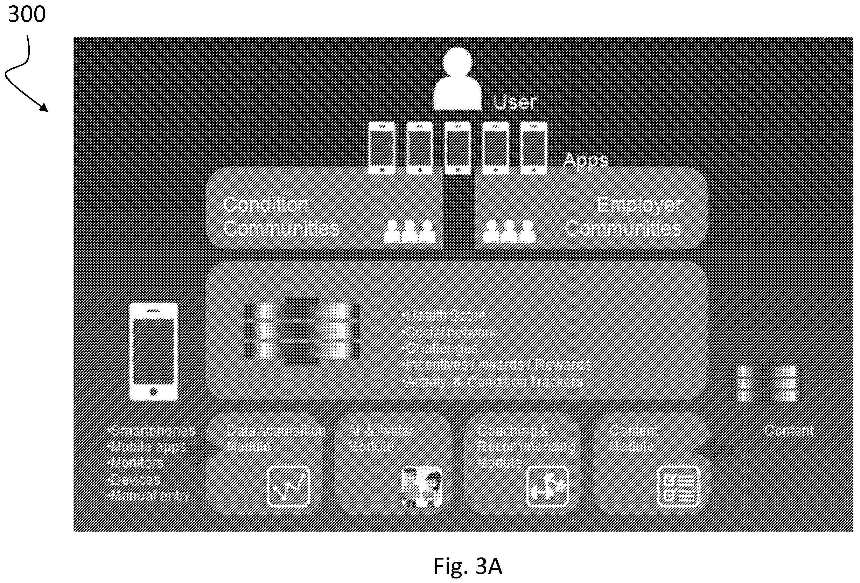

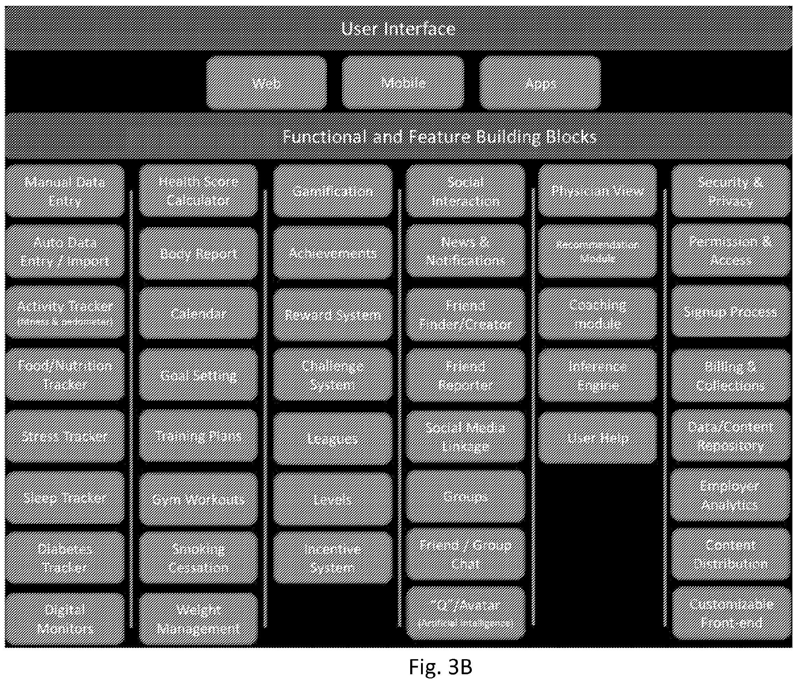

Fig. 3A is a block diagram that illustrates functional building blocks 300 associated with a health platform, including for calculating a health score, as well as implementing many of the features shown and described herein. The health platform system in accordance with the present application can be accessed via Internet web browser software applications (e.g., CHROME, FIREFOX, SAFARI, INTERNET EXPLORER), and by using a desktop or laptop computer as well as from a mobile device, such as a Smartphone or Tablet via a mobile optimized version of the web site. An implementation is illustrated in Fig. 3B.

The health platform 100 can be configured with a smartphone software application, referred to herein generally, as the "tracker application," to track fitness activities in an easy and automatic way (in addition to providing for manual entry) and the recorded/tracked activities can be uploaded automatically on the health platform. The tracker application can be provided for devices operating IOS, Android and BlackBerry operating systems, and can be provided at no charge to the user.

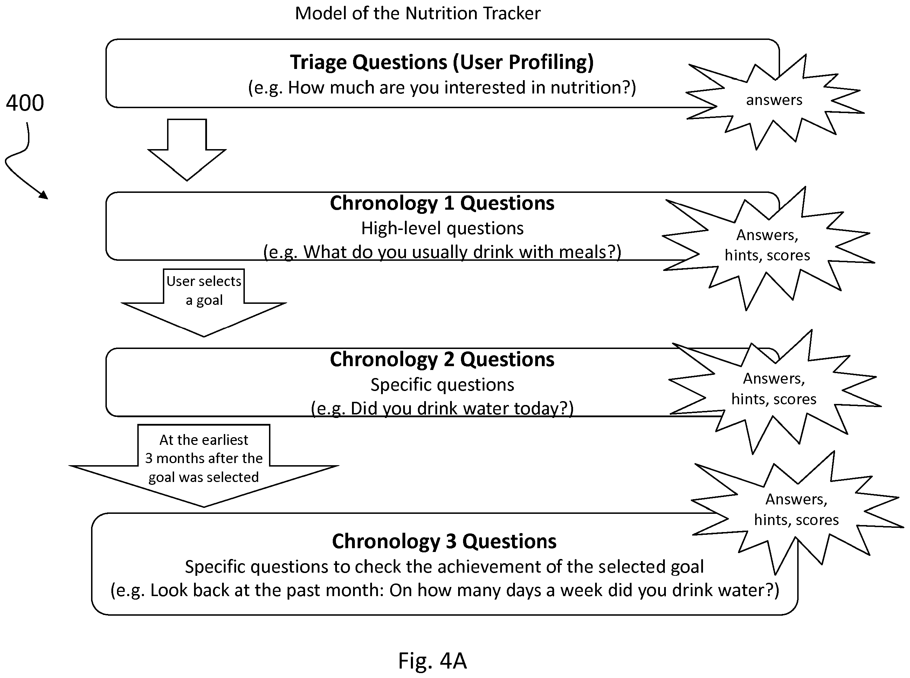

An example flowchart illustrating example steps 400 associated with nutrition tracking is illustrated in Fig. 4A, Example steps include asking and receiving responses to questions associated with a user's interest in nutrition, goals and progress, and a plurality of chronology questions.

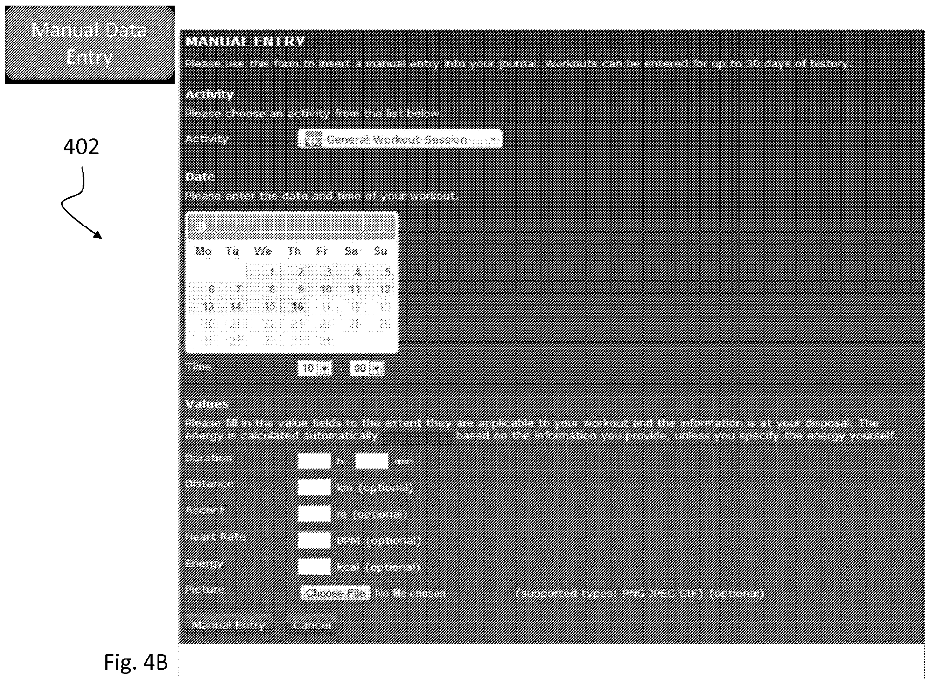

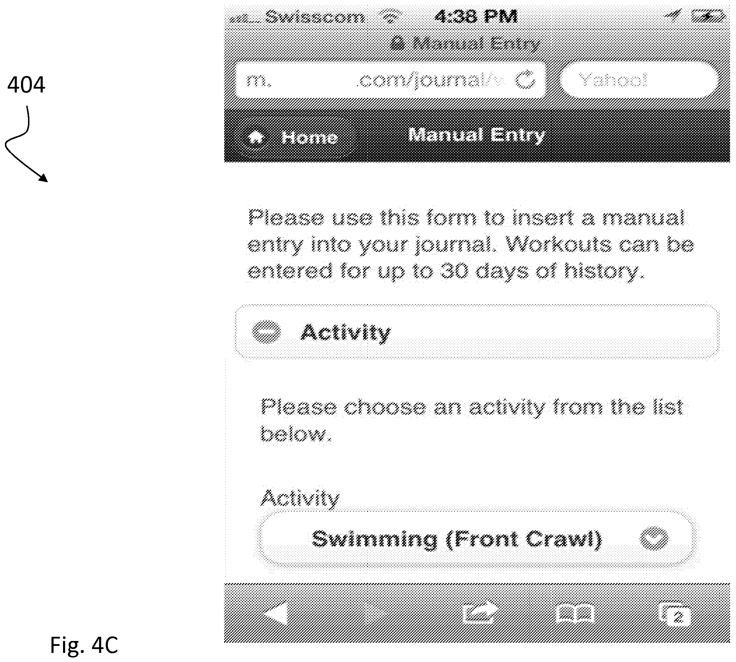

Figs. 4B and 4C, illustrate example screen displays 402 and 404 associated with manually entering data, e.g., via a graphical user interface via screen controls (e.g., buttons, icons, drop-down lists, radio buttons, checkboxes, textboxes or the like) and submitted by the user. As shown in Figs, 4B and 4C, information, such as relating to indoor and outdoor activity can be inserted manually via a web form (Fig. 4B) or via a mobile platform (Fig. 4C) and users can also choose to upload images together the information associated with their activity.

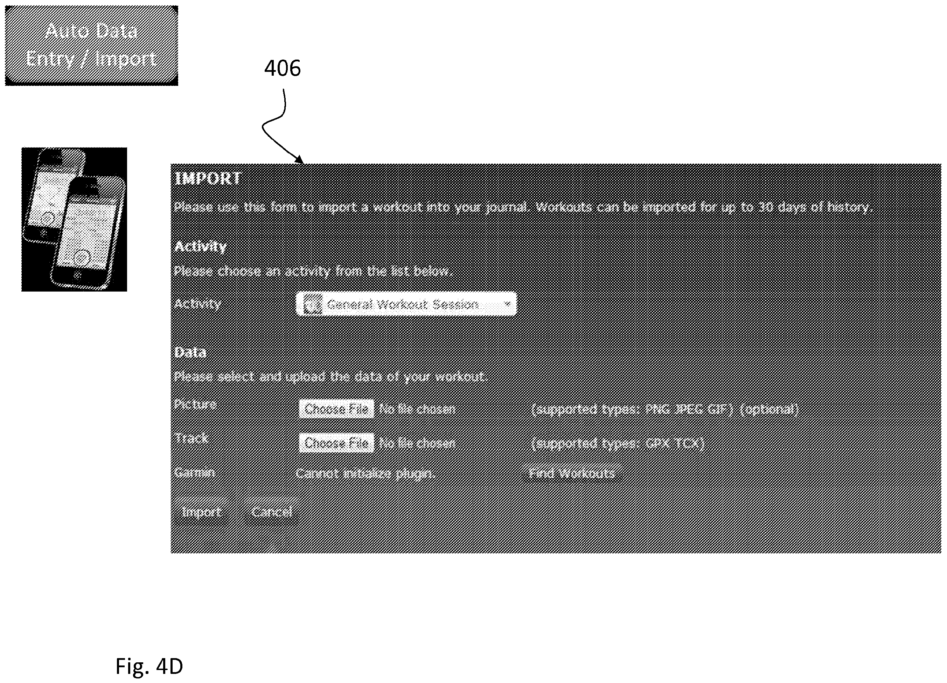

Alternatively (or in addition), data entry can occur substantially automatically, such as via an import process of one or more tiles formatted in one of various file types (e.g., TXT, DOC, PNG, JPEG, GIF, GPX, and TCX). Fig. 4D illustrates an example data entry display screen 406 that is provided to a user for importing data associated with a particular activity via the tracker application. In the example display screen 406, workout data are uploaded via the tracker application.

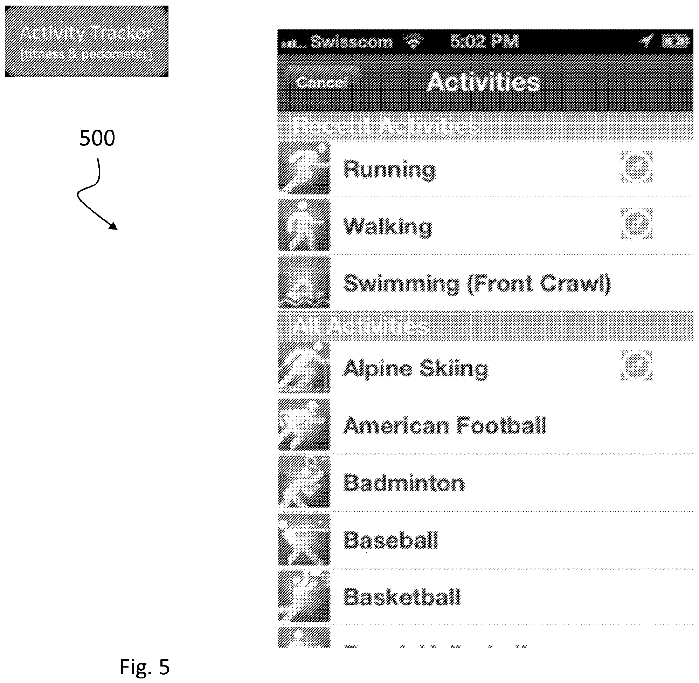

In one or more implementations, the present application offers the tracker application to track a user's fitness activity, and can be implemented on devices running IOS, ANDROID, WINDOWS PHONE, BLACKBERRY and other suitable mobile device operating systems. Outdoor and indoor activities can be tracked, and data upload to a server computer or other device can be provided in a secure format. The data can be seamlessly and automatically integrated for calculating a user's health score. For example, daily activity measured by stepcounters/pedometers or other similar devices can be integrated using the systems and methods shown and described herein. An example and tion-exhaustive list of activities provided via the tracker application and usable to calculate a user's health score is illustrated in an example display screen 500 in Fig. 5,

In one or more implementations, a plurality of integration strategies are supported to integrated. For example, server-side integration can be employed to integrate devices. Alternatively, mobile integration can be supported, which integrates devices into the tracker application (or other suitable mobile application). Health data can be organized per user, and can be provided in connection with: body dimensions (height, waist circumference); body weight (including body fat); blood pressure (including pulse); blood sugar levels (fasting flood glucose); blood lipids (total, high-density, low-density, triglycerides); and workouts (duration, distance, ascent, descent, velocity, energy, trackpoints, heart rate, pictures).

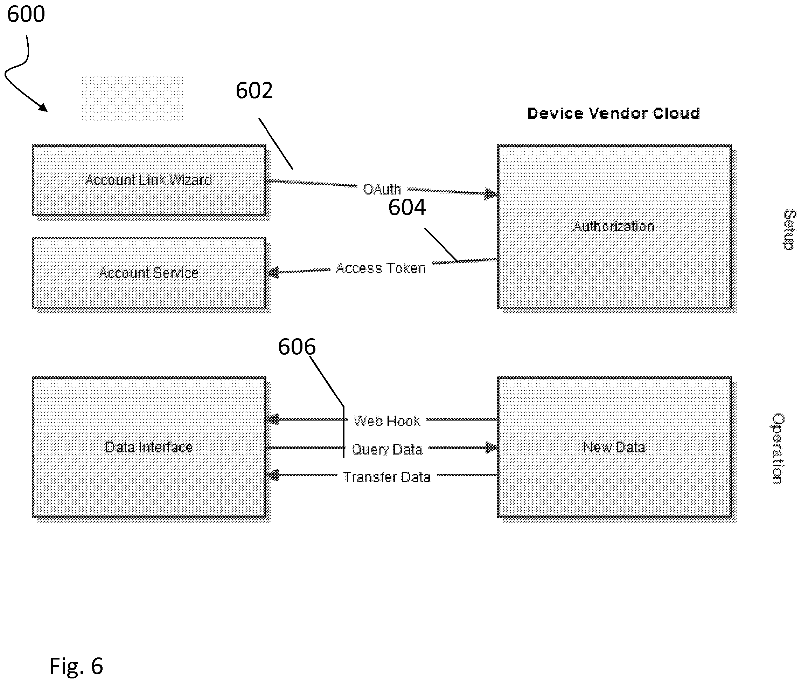

Fig. 6 is a flowchart illustrating steps 600 for server-side integration of a device in accordance with one or more implementations of the present application. After a decision is made deciding in what journal to place the data, an account link wizard is implemented that allows users to connect their account(s) to a cloud account, which can be provided by a device vendor. This connection can be created using a suitable standard, such as OAuth (step 602). In case the cloud account contains data from multiple users in a home, a single user profile can be selected as part of the connection step. Further a data interface can be developed. Once an account link is established, the cloud can execute a web hook whenever new data becomes available. That data can be pulled from the cloud using security credentials, such as via an access token (step 604). In order to facilitate implementation of the above-identified steps, a generic account service can be provided that allows for managing links to external accounts on a per-user basis and in a sate and efficient way. Periodic account operations, such as subscription/web hook renewal, and one-time operations, such as asynchronous bulk data loading, can also be supported. Example technical features can include: HTTPS, RESTful (service model), OAuth (authorization), JSON or XML. (data format) and Web Hook (new data notification) (step 606). This infrastructure enables prompt and efficient integration of new devices.

With regard to mobile device integration, sensors that can be attached to a mobile device can often be integrated by the user uploading sensor data, e.g., to a cloud device using a mobile phone app. A server or other computing device can receive sensor data from that cloud device via server-side integration, as described above. A direct integration of devices into the mobile app in accordance with the present application can be suitable in connection with partial information (e.g., heart rate to be correlated with a workout being tracked), data confidentiality (e.g., data directly sent and not passed through a cloud device). and ease of use (e.g., by reducing the number of user accounts needed for implementation of the presentation).

Integrating a device, such as a sensor, directly into the tracker application can include support for iOS, Android, and/or BlackBerry, Windows Phone operating systems. Other support, such as provided via library files, can include operation to check for the presence of the sensor; operation to read current sensor data; support for operation to pair with the sensor; callbacks on relevant events (new data, peak detected, etc.), capability of supporting multiple applications using a library concurrently, and capability of operating when the application is in the background.

In one or more implementations, a food/nutrition tracker feature is proved that provides a single score (with sub-scores), as well as being scientifically founded, being applicable internationally, and includes quantitative and qualitative data (e.g., amount and type of food/beverage). In one or more implementations, the food/nutrition tracker feature is easy to use (e.g., via "two clicks"), user-friendly, fun, attractive, sexy, and motivating instead of moralizing. In one or more implementations, the food/nutrition tracker feature includes learning, such as by tracking how a user behaves, and is individualized to customize how the program responds. The focus of th e food/nutrition tracker feature is on a healthy diet and favorable eating behavior. Moreover, the food/nutrition tracker feature can focus on sustained weight management rather than weight reduction. Thus the tracker is not merely a calorie counting application, but rather prompts the user towards healthier options at mealtimes. Moreover, and as noted above, information associated with the food/nutrition tracker feature is integrated seamlessly and substantially automatically for calculation of the user's health score.

As noted herein, an individual's health depends on various interrelated factors. One important determinant of health is lifesty le. The physical, social and occupational environment of people largely defines the general framework for behavior, particularly when it comes to health. Notwithstanding the environment, a person's health substantially depends on the everyday choices made towards promoting health behavior and how to resist behavior that is hazardous.

The present application focuses on four domains that not only have a strong impact on health, but can also be improved. The domains include 1) physical activity, 2) stress, 3) sleep and 4) diet. The food/nutrition tracker feature focuses on health improvement through healthy diet and nutrition.

It is recognized that energy and nutrients in food and drink, for example, directly impact risk factors e.g., blood lipids, as well as the risk of a heart attack, stroke, cancer and other non-communicable diseases. Similarly, an immoderation of calories can lead to weight gain. Excessive body fat can excrete hormones or modify or impair the effectiveness of hormones and increase risk factors, such as high blood pressure or unfavorable blood lipids, While food composition is important, so is energy balance. The way people eat is a result of fixed patterns, which makes eating behavior resistant to change. It is, therefore, unlikely that following a simple program such as dieting or counting calories will lead to sustainable behavioral changes in the majority of cases. When, why and how a person eats need to be addressed in greater depth.

In order to be able to determine an individual's potential for improvement, specific behaviors need to be examined and, if necessary, adapted. Many adults have behavioral patterns that have been stable for years. For a change to become sustainable, selected improvements in nutrition and eating behavior have to fit to an individual's lifestyle and have to steadily become a part of it. The food/nutrition tracker feature of the present application addresses this by prompting a suggesting a selection of potential improvements to the user that are customized to the user's own nutrition and eating behaviors.

In one or more implementations, the present application enables users to sustain positive lifestyle changes. The food/nutrition feature of the tracker application can take into account various aspects, including sustainability with respect to body weight. The food/nutrition tracker feature processes information to enable a user to sustain a healthy lifestyle, and avoid promoting quick fixes, for example, for weight loss. Users receive information to manage body weight in order to achieve a healthy body weight and avoid weight gain. For users who wish to lose weight, scoring can be adapted to focus on energy balance. Moreover, a weight management module can be provided that prompts for specific weight-related questions about diet and eating behavior, and that processes information received in response to the prompts to provide tailored and specific hints.

In one or more implementations, the present application enables users to improve and/or strengthen health resources. Such resources allow people to maintain their health status and to better cope with potentially hazardous influences, such as disease risk factors (as described herein).

In addition, a food and beverage intake component can be included in food/nutrition tracker feature, and can be relate to the MEDITERRANEAN DIET (MD). Adherence to the MD is believed to result in an improvement of risk factors such as insulin resistance, high blood pressure and blood sugar or impaired blood lipids. Eating and drinking according to the MD is also associated with a reduction in morbidity and mortality of major chronic diseases, including cardiovascular disease, cancer, diabetes, Alzheimer's and Parkinson's disease.

It is recognized than an advantage of the MD is that it is easy to follow. The MD can be administered in all western cultures. In general, dishes are easy to prepare and ingredients are readily available and affordable. Furthermore, the MD penetrates restaurants and canteens more and more. Finally, the MD is tasteful, variable and appealing. Scientifically, the MD provides the basis for an ideal approach to healthy eating and drinking, and offers an excellent probability of users sustaining a desired and/or healthy body weight.

In one or more implementations, monitoring and maintaining positive eating habits are a substantial element of the food/nutrition tracker feature. The food/nutrition tracker feature can pose questions about a user's eating habits in order to detect problematic eating behavior, with focus on breakfast habits, meal circumstances (e.g., eating alone or in company) and duration, frequency and regularity of meals, snacking, as well as eating out, eating while doing other activities e.g., watching TV, cooking and preparation of meals, shopping for food and "emotional" eating. The latter occurs when people do not eat because they are hungry or have appetite but because of emotions such as stress, frustration, loneliness, lack of sleep or physical activity.

Thus, in one or more implementations the food/nutrition tracker feature helps users to keep their weight on track, and supports those who want to lose weight. For example the food/nutrition tracker feature can assist users with strict scoring, guiding the user towards a lower caloric intake. In one or more implementations, the food/nutrition tracker feature stresses reasonable weight reduction and maintaining a lower body weight. The food/nutrition tracker feature can target sustainable lifestyle changes by using more specific questions and tailored, practical prompts.

In one or more implementations, the food/nutrition tracker feature of the present application can be implemented in conjunction with a rule engine ensuring that feedback can be modified in mostly declarative ways, requiring little programming. In addition, various communication channels, such as a web channel, an e-mail channel and mobile app channels are supported. Moreover, user profiling is provided, and one or more questions are provided, such as regarding the user's dietary avoidances, interest in nutrition and occupational status. The food/nutrition tracker feature of the present application covers the following domains (qualitative and quantitative): 1) food intake, 2) beverage intake and 3) eating habits. These three domains can be further subdivided into sub-domains.

The food/nutrition tracker feature of the present application precludes repetitive prompts to avoid boring and/or jeopardizing the user's interest. For example, the food/nutrition tracker feature starts off in a high-level way, such as by asking the user questions about his/her typical consumption behavior, such as "Do you drink water with your meals?" Based on the answers received from the user, the food/nutrition tracker feature may provide increasingly specific questions about the user's consumption and behavior, such as, "Did you drink water today?"

The food/nutrition tracker feature of the present application can also include different types of questions, such as yes/no questions, selection questions (single choice, multiple choice) and value entry questions, In an implementation in connection with a mobile computing device, a user interface can be optimized for touch operation, e.g., using large check boxes, large selection buttons, and sliders for range-based input. The labeling of sliders is generally based on the local unit system of the user, whereas the valuation rules can be based on S1 units. The user interface ensures the proper translations and representation of values. The user's answers allow the food/nutrition tracker feature of the present application to monitor the progress of the user in achieving self-set goals.

The food/nutrition tracker feature of the present application can alternate questions randomly, between domains and sub-domains and not in a fixed order. Furthermore, the food/nutrition tracker feature of the present application also prompts questions depending on particular context (e.g., depending on the time of the day), thereby reducing lag time between an event and its recording. Some questions can be asked on specific days, e.g., on Sundays, Further, questions that the user does not answer can be asked again after three to four days, and can be repeated again if the user still does not answer. In an implementation, if the user does not answer 10 consecutive questions, the food/nutrition tracker feature of the present application can prompt the user to resume.

It is recognized that goals for dietary achievement should be realistic, particular from the individual point of view of the user If too many goals are imposed that are unachievable or unstructured, the user will become frustrating and confused and thus become counterproductive. The food/nutrition tracker feature of the present, application avoids this by proceeding methodically, first getting to know the habits of the user and then detecting areas willi potential for improvement. Based on the information obtained from the user, the program defines realistic goals, which are suggested to the user and ordered by priority. The program can then ask the user which of the three goals he/she wishes to achieve first. The food/nutrition tracker feature of the present application can follow a step-by-step approach, meaning that goals need to be worked on by the user (from fully achieved to not achieved or postponed) before new goals can be suggested. Thus, the user works on only one goal at a time. Once a week, for example, the user decides if he/she wants to continue working on the goal, work on another goal or take a break from working on goals.

In an effort to keep a user motivated, rewards may be provided when goals are achieved. Besides virtual rewards, such as medals, cups or titles, competitive elements can be used to increase positive feedback by the food/nutrition tracker feature of the present application. Further rewards can include special treats (e.g., free entry to the gym for a month).

In one or more implementations, responses to prompts can trigger one or more specific hints. Hints are aimed at leading the user towards achieving a goal, either supporting the user to make healthier choices in the future or praising the user for his/her healthy behavior. Hints provide not only concrete instructions, but also the rationale behind them, This increases user motivation and adherence to the program. Some responses can be followed questions immediately, before a hint is given. In one or more implementations, hints are provided by an avatar. Referred to and shown herein, generally, as "Q," the avatar can communicate in the first person singular form (e.g., "May I make a suggestion?"), which aims to create a personal relationship between the user and the food/nutrition tracker feature of the present application.

In response to prompts from the food/nutrition tracker feature, a score can be attributed that can include three dimensions: 1) favorable behavior, 2) indifferent behavior, and 3) unfavorable behavior. A corresponding score can then be factored with one or more other nutrition-related scores, and applied in the calculation of a user's overall health score. For example, a nutrition-related score can be calculated with a sports-related tracking score originating from physical activity, a stress score, a sleep score or the like. Moreover, in one or more implementations, the present application supports transparency in that the user has access to his/her scores at any time.

In one or more implementations, the tracker application captures stress-based information, based on the data acquired, for example, via sensors on smartphones and questionnaires. In one or more implementations, heart rate variability (HRV) can be monitored with an integrated external heart rate band. Alternatively, sensors may be implanted in a body, such as a pacemaker or other technology, that is operable to transmit information to a computing device. In one or more implementations, the sensors that are provided in accordance with the present patent application can be non-invasive or invasive. For example, the sensor(s) can detect heartbeats and can provide for transmitting data from an implanted pacemaker. Alternatively, blood sensors that are mounted in a person's body transmit data, for example, to detect one or more marker proteins that may be present in the wearer's blood. Thus, the present application is usable with one or more sensors that are placed in or with the wearer's body, and/or are otherwise configured to communicate with devices that are implanted in a person.

In addition, the device can be configured to detect and/or display humidity associated with user's skin surface. Humidity information is usable, for example, to detect that the user is or is getting dehydrated and should drink. In one or more other implementations, DNA information and/or one or more biomarkers is accessible, for example, to examine biological processes, pathogenic processes, or pharmacologic responses, such as associated with one or more therapies.

In one or more implementations, the stress tracker allows the user to enable/disable stress tracking, with controls for recording of voice, social, and movement, stress. The user's current stress score can be displayed, and can allow a user to start an overnight HRV measurement session. Moreover, the stress tracker can show the result of the overnight I-IRV measurement session, and asynchronous/ interactions with the avatar ("Q") can be shown. For example, the avatar "Q" can ask the user for a voice sampling. Moreover, the avatar "Q" can ask the user to answer one or more specific question sets. The avatar "Q" can further recommend to the user to do an overnight HRV measurement session. In addition, information obtained thereby can be seamlessly and substantially automatically integrated into the user's health score. In addition, sleep tracking can be provided in a mobile application, implementation of the present application. For example, a seamless integration of Heart Rate Variability and diagnostics of sleeping patterns. See, for example, the example shown in Fig. 6.

In one or implementations, a plurality of monitoring devices can be employed that use various operating systems and/or platforms. One or more application programming interfaces ("API's") can be provided to support integration and comunication among and between various kinds and brands of devices.

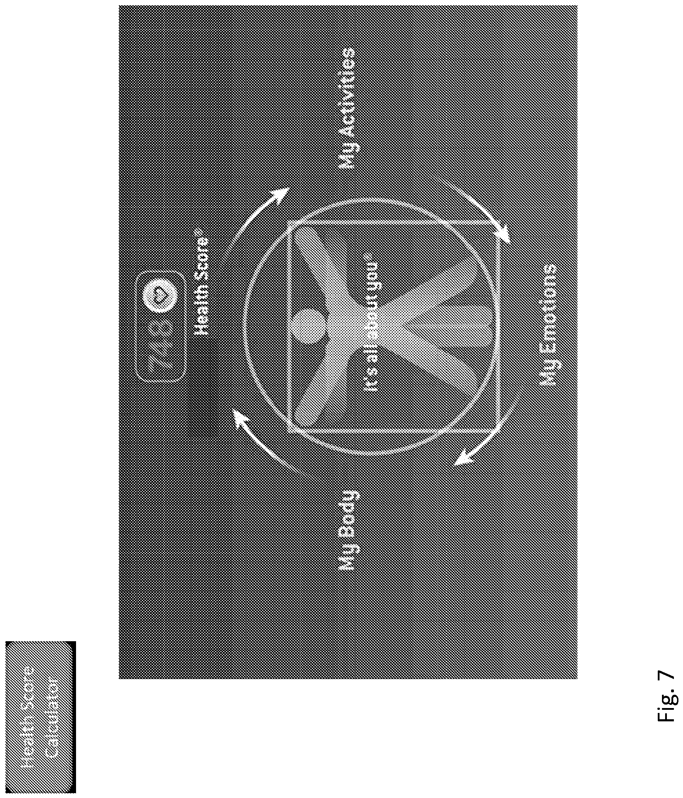

With reference now to Fig. 7, the present application can calculate a personal health score for each of a plurality of persons, which can be represented by a number from 1 (representing a poor score) to 1,000 (representing a excellent score), and can be provided current health and fitness status information substantially in real-time. When tracked over time, the health score offers a directional relative indicator of how a user's health and fitness is improving or deteriorating. In this way, the health score provides output substantially in teal-time and provides a virtual "mirror" of the user's overall health and fitness. This provides an avenue for the user to maintain health & fitness awareness level high. Furthermore, with the introduction of a score, a user can benchmark him or herself against others, all the time.

The health score of the present application can be analogized as Celsius/Fahrenheit to measure temperature. Rather than describing temperature in terms of 'cold' or 'warm,' for instance, temperature can be precisely and numerically represented. Similarly, the health score of the present application is useful to precisely and/or numerically represent a person's health. Moreover, in one or more implementations, a "what if" scenario can be provided for users to enter one or more variables to determine how various behaviors can affect a user's health score (e.g., quit smoking, losing weight, etc.). Moreover, in one or more implementations, the health score of the present application factors three values representing the following categories of information received from a user: who the user is, which can include description of the user; how the user feels (such as emotions, quality of life, etc.); and what the user does (such as activities, lifestyle components, etc.). The health score in accordance with the present application can represent a "living score," one that is dynamic and learning over time. With the introduction of new information from the user, and new medical breakthroughs and developments, the algorithm can be optimized over time.

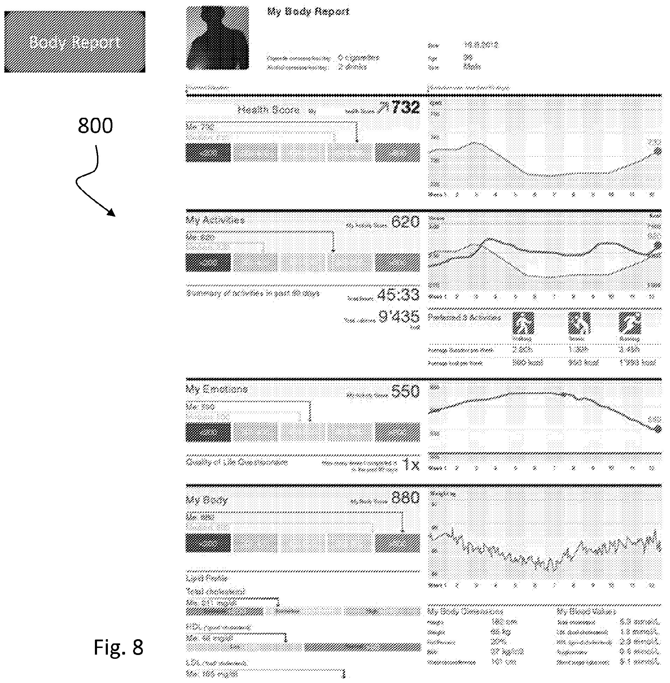

Fig. 8 illustrates an example body report 800, divided in accordance with an implementation of the present application. In the example shown in Fig. 8, the body report 800 is formatted as a 1-page report that includes the key data on the user with regard to his/her health score, as well as sub components both currently and over time. The report can be useful for personal use, or be shared with a personal trainer or a health professional, for example, in case the user chooses to share it.

In the example shown in Fig 8, a 36-year-old male reports smoking 0 cigarettes and consuming 2 alcoholic drinks per day. The user's health score indicates improvement, shown by an arrow rising next to the user's health score of 732. In addition, the example body report shows the users health score graphically presented over a 12-week period. The example body report shown in Fig 8 also includes the user's activity numerical score, which is also graphically represented over a 12-week period. Additionally comparative data can be provided both in terms of actual number values (e.g., the user score versus median scores), as well as graphically, including a plurality ofcolored rectangular portions representing ranges of score values, and where the user lies therein. Other information represented in the body report includes values associated with the user's emotions, and kill overall body score, which can be similarly represented numerically and graphically.

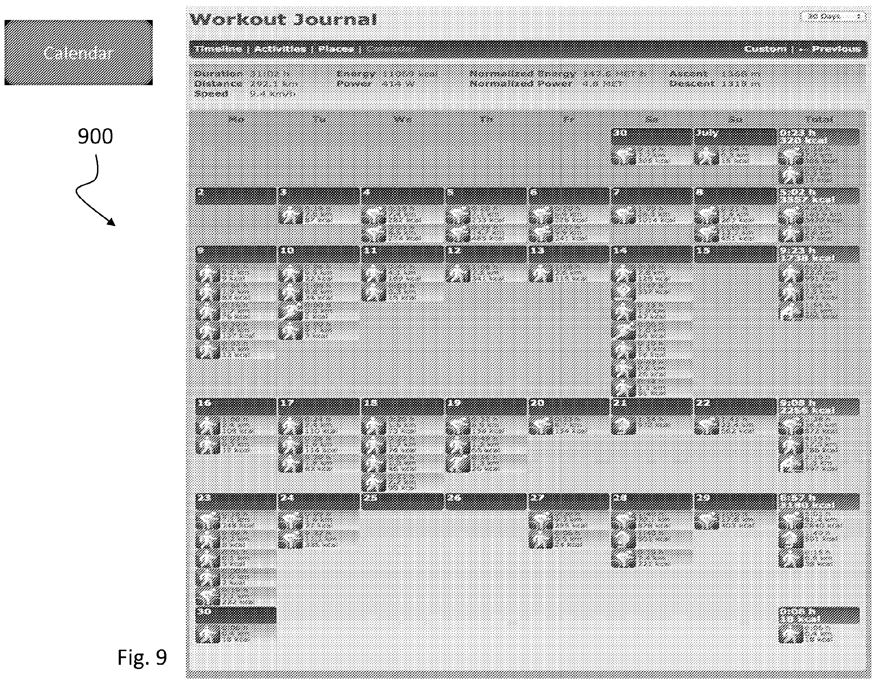

Fig. 9 illustrates an example display screen 900 that provides a calendar view for users to review their fitness activities and receive feedback data on weekly/monthly hours trained and calories burned by activity and as a total. In the example shown in Fig. 9, a calendar view is provided that allows users to visualize training plans, challenges, and activities, and to export calendar data to one or more email client applications, such as MS-OUTLOOK.

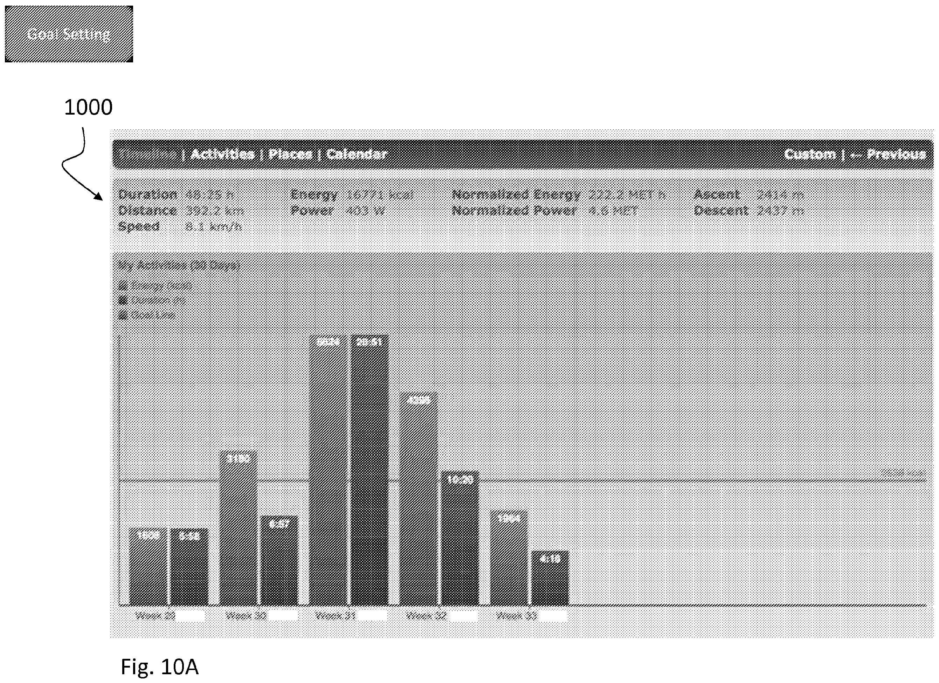

With reference now to Fig. 10A, a display screen 1000 is provided that includes a graphical indication of a user's goals activities, both in terms of energy and duration. Moreover, a goal line is provided in display screen 1000, which provides the user with an amount of calories he/she needs to bum per time period to maintain the user's current health score. In connection with certain features associated with goals, goals can be set by both users and health professionals, and can span a wide range from simple goals over training plans to specific programs. In one or more implementations, a goals catalog can be included for a user to select one or more goals. Examples include, workouts (Burn $n$ energy per week for $t$ period target date, log $n$ activities per period, run a marathon by $t$ date, etc.), health score (reach a score of "$n$" by target date, etc.), Other examples include: training plan; achievements (complete achievement a by target date, etc.), smoking cessation program; and weight management.

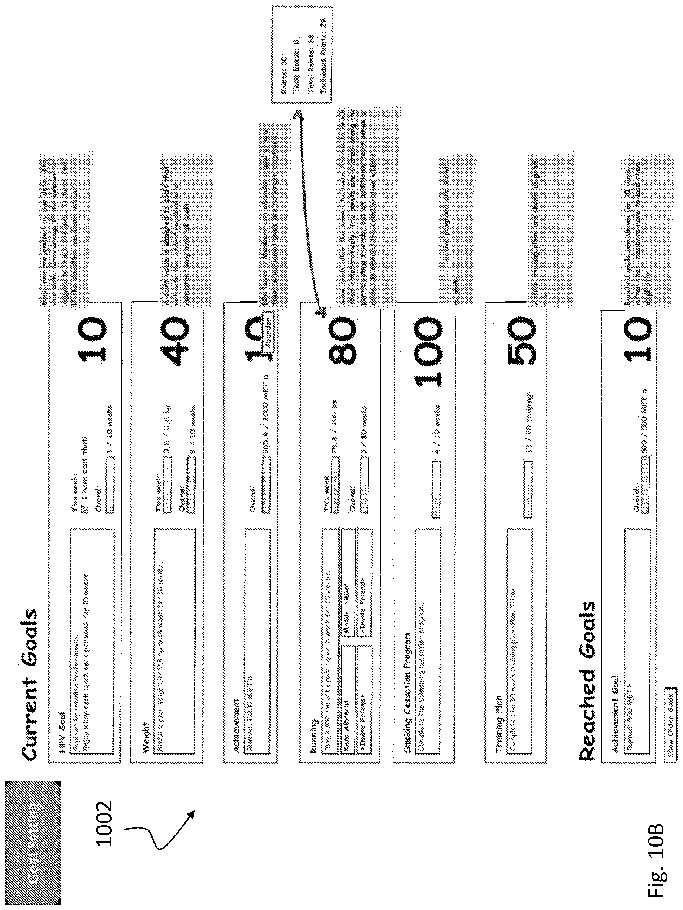

Fig. 10B illustrates an example goals page display screen 1002 associated with current goals and reached goals for a user. The goals page display screen 1002 in Fig. 10B shows current goals of a user, and as shown in the example in Fig. 10B, each goal is listed with a visually strong percentage bar, showcasing the progress made towards reaching the goal. For each goal, an indication can be provided whether, based on the current progress, the user is leading or lagging with regard to the target date. As noted herein, goals can be set both by users and health professionals, including via a user interface for health professionals. Goals can span a wide range from simple goals over training plans to specific programs, and goals can have respective target dates. The present application guides users from the health score drivers (e.g., via health score Refactoring) to specific goals, such as via particular programs.

A goals catalog can be defined for a user. For example, a goals catalog can include one or more of the following features. Workouts: Burn $n$ energy per week for t weeks/months by target date: track $n$ of metric m per week with activity $a$ for t weeks (e.g., 25 km of running) log $n$ activities per week for $t$ weeks, health score: reach a health score of $n$ by target date; reach a health reservoir score of $n$ by target date; maintain a health reservoir score above l for $t$ weeks. Journals: reduce metric $m$ by $d$ every week for $t$ weeks (e.g., weight); reduce metric $m$ to $n$ by target date (e.g., blood sugar or lipids). Training Plan. Achievements: complete achievement $a$ by target date. Smoking Cessation Program: information; questionnaires; notifications. Mediterranean Diet Program: information; and daily recipe notifications.

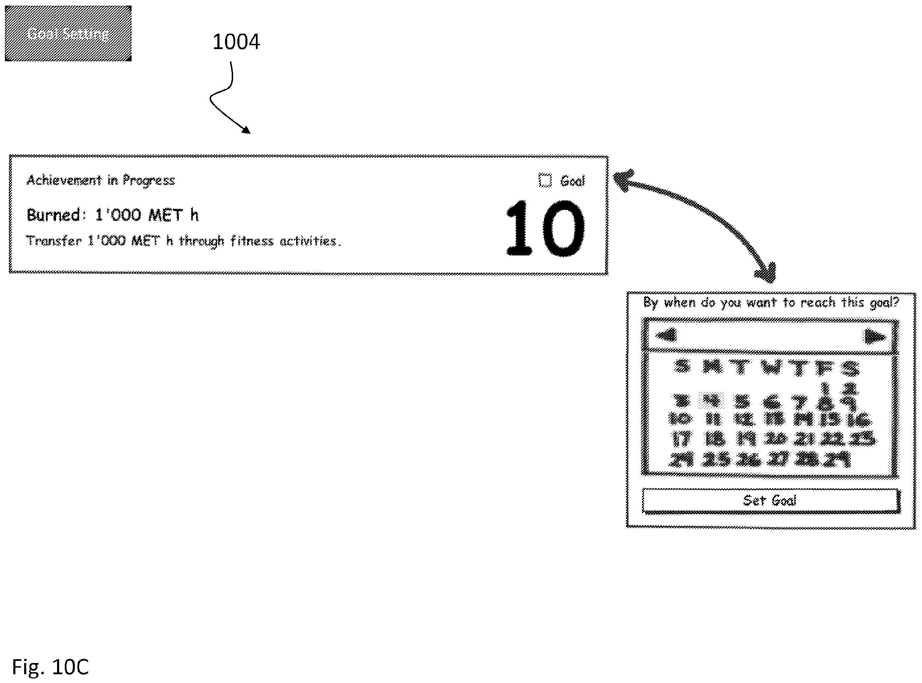

Goals can be defined, such as by user and/or healthcare professionals at various points or places in connection with the present application. An example goal definition interface 1004 is illustrated in Fig. 10C.

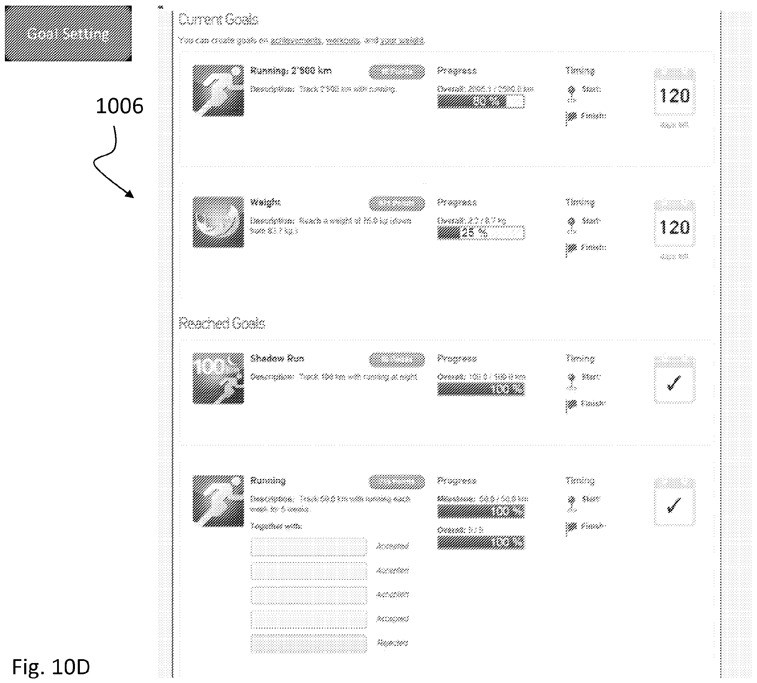

Fig. 10D illustrates an example display screen 1006 enabling creation ofgoals as a function of achievements, workouts and weight.

The present application also supports the development and monitoring of training plans that can include providing entries specifying detailed workouts. Workouts can include time, duration, energy, mood, as well as the warm-up, cardio, core, resistance and cool-down phase, and information of each of which can be captured. Various activities can include cardio exercises on fitness machines, as well as other types of activity, such as running, cycling, fitness classes, and review session(s) with a personal trainer. In connection with a workout, users can note changes to sets and repetitions given in the plan on their mobile devices. Also, the mood for the workout can be logged. Training plans can be edited by drag and drop and copying entries from one weekday to another, and copying entire weeks to another week. Users can be able to print individual training plati entries as well, such as for taking to the gym.

Moreover, one or more gymnasium workout models can be provided for popular gym classes, such as Zumba, Body Toning, or Body Pump. In one or more implementations, automated integration of user's gym classes is supported, for example, and can include a heart rate tracking algorithm. For example, nano based/plaster sensors can be integrated with the teachings herein.

In one or more implementations, the present application employs gamification, which refers, generally, to the use of game design techniques, game thinking and game mechanics in non-game contexts. Gamification is used to make technology more engaging, by encouraging users to engage in desired behaviors, by showing a path to mastery, by helping to solve problems, and by taking advantage of a person's psychological predisposition to engage in gaming. Applying these principles in a health & lifestyle context makes for a powerful end user experience. By employing gamification, behavior can change, which represents a huge opportunity to improve health outcomes. Moreover, the combination of mobile technologies with social networking and gamification principles has the power to facilitate healthy lifestyle behavior change in individuals. Accordingly, the present application can apply gamification principles in various ways across the platform to engage users and encourage them to adopt a healthier lifestyle, which includes but is not limited to: Achievements; Rewards; Challenges; Leagues and Levels.

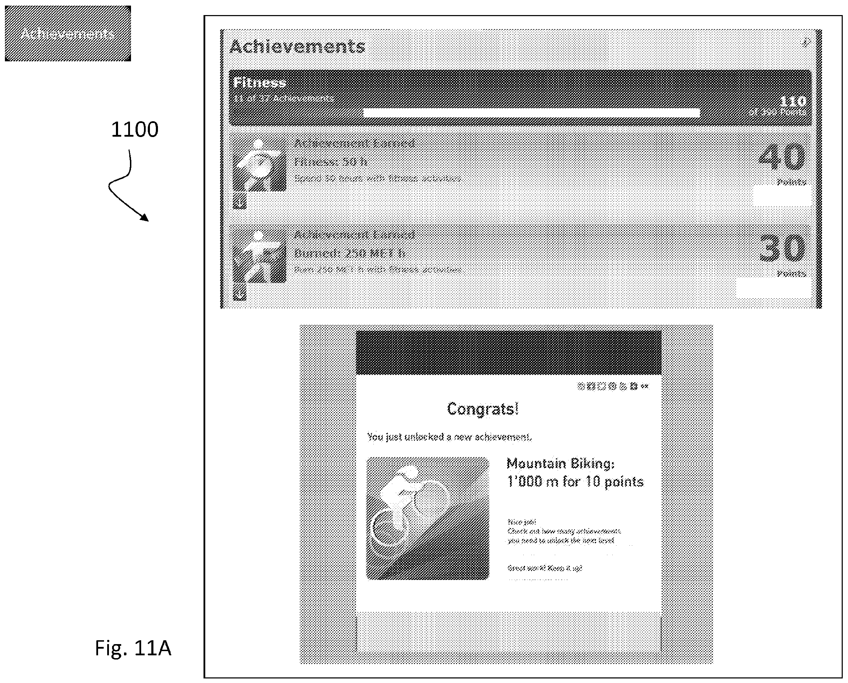

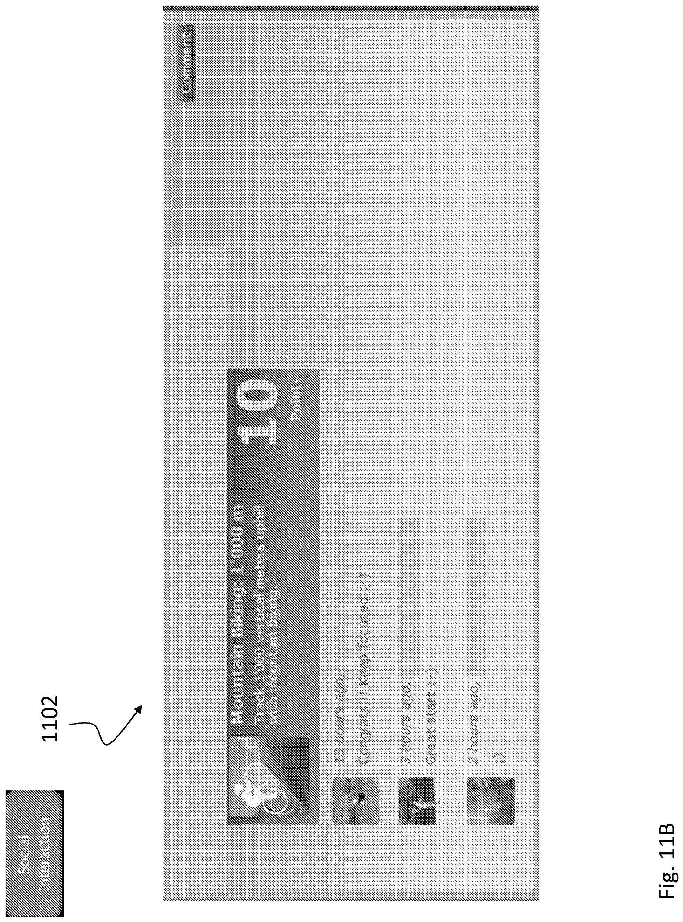

Fig. 11A illustrates an example display screen 1100 associated with recognizing achievement and providing awards for user progress. By providing achievements and reward tracking in specific activities, the present application provides "pat on the back" feedback, which encourages users, such as by saying "congratulations" or "well donel" Messages can be provided graphically (e.g., trophies and awards), or with language. In one or more implementations, achievement messages can appear in a newsfeed, such as on a user's social network home page, which be shared via social media such as Facebook and Twitter. In connection with social networking, the present application includes interaction/interface with a user's newsfeed, commenting (such as on news items, achievements, activities), forums/-discussions, picture sharing, video sharing, platform notifications, and push notifications. Fig. 11B illustrates an example display screen 1102 that demonstrates social interaction, which can be implemented by providing a medium for users to comment on each other's activities, including by supporting user sharing of multiple photos and activity events.

In connection with achievements earned, the present application provides "gamification" points, which can be awarded for motivation and reward purposes. In one or more implementations, achievement points are not factored into a user's health score. For certain achievements additional rewards can be earned that include, for example: a title that can be displayed on a user's profile; a pin that can be displayed next to a user's profile, or a special "wallpaper" that can be downloaded and used on a user's desktop or smartphone.

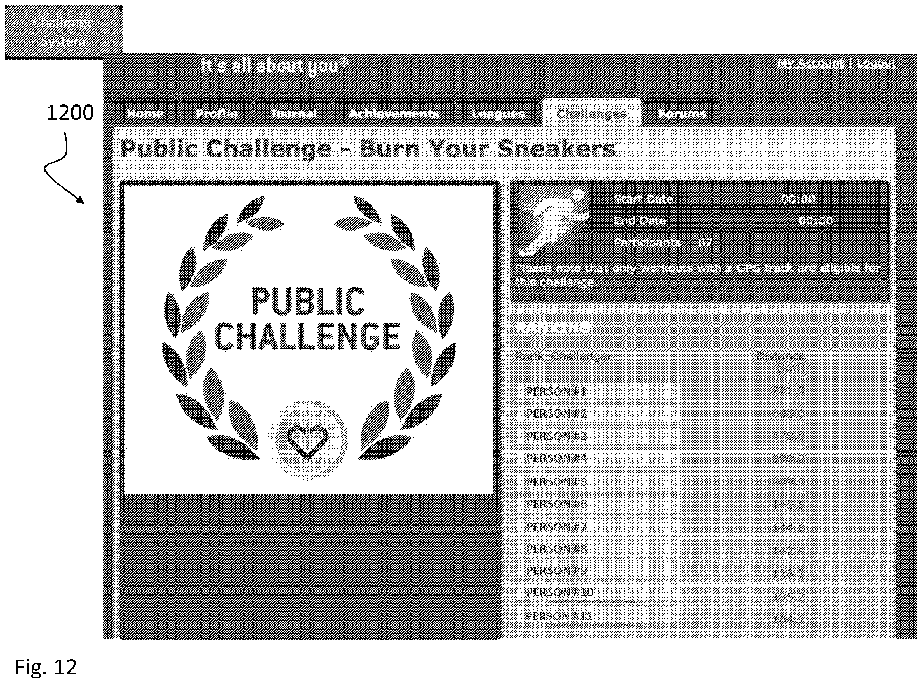

In one or more implementations, challenges can be supported that provide a direct way for users to compete with other users on a Health platform 100 in accordance with one or more implementations. A challenge system can provided for various people or groups, such as individuals, groups, corporations, fitness clubs and public use. Individuals can use the challenge system to compete with their immediate friends. Groups can use the system to issue group wide challenges to their users and public challenges and/or a corporate customer and all platform users have the opportunity to compete in their challenge of choice. Further, team challenges can be supported that allow for teams to compete against each other (e.g., marketing versus sales department or a given client company). Group challenges and departmental specific challenges within corporations can be useful to create motivational activity. An example public challenge display screen 1200 is shown in Fig. 12.

Further, leagues can be supported that can engage users in more direct competition than achievements, but can represent less direct competition than challenges. In connection with leagues in one or more implementations, a user completes three workouts in a specific fitness activity to qualify for a league. Leagues can be broken down by type (Bronze, Silver, Gold and Platinum), activity type and division. In one or more implementations, leagues can run over seasons that last weeks to several months. Achievements and rewards can be linked to the league system, and league promotions can be shown in a user's social network tiews-feed. Achievements for promotion to a higher league can be earned, including for finishing a league season in top ranked positions.

The present application provides support for levels, incentives, and social interaction. Progression dynamics in form of Level-Systems can be integrated in the Health platform 100, substantially as shown and described herein. Various features can include a seniority level based system. For example, a new user starts at Level 1 and gradually progresses and rises in levels along the way. Levels can be determined by the number and kinds of activity points that a user has earned. Activity points can be rewarded for tracking workouts, earning achievements, and commenting on news items.

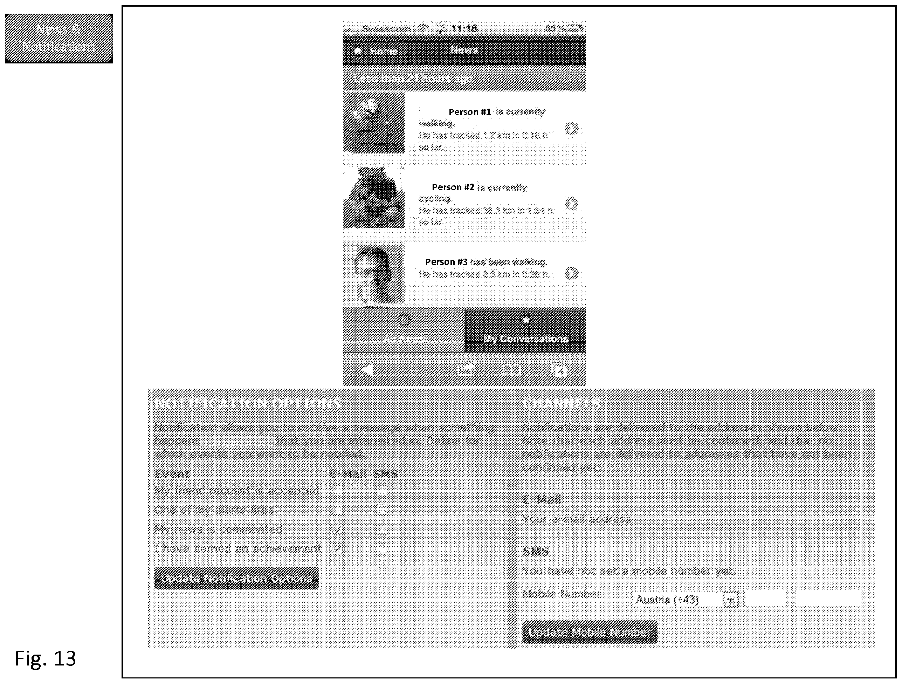

Referring to Fig. 13, news and notifications provided in accordance with the present application include a "Newsfeed" (as known in the art) posting, notifications (e.g., by SMS or Email), and platform notifications (e.g., using graphical controls shown and described herein). In one or more implementations, a "Newsfeed" provides users with recent activity information of their friends. Users can choose to receive notifications about what is happening, for example, via SMS or email. Moreover, push notifications to the devices can be provided as well. SMS notifications can be useful for users who want real-time encouragement from their friends while the users are out training. Other social networking functionality is provided, such as for finding friends for new introductions or to reconnect with others. A friend finding feature can be provided for users to friend other users on the system via a name search or email invite, or can use an integrated fitness style search that can include both public events and sports style search, e.g., "I'm a runner looking for other runners." Moreover, a user-friendly friend reporter system can be provided, for example, via a Newsfeed to keep users notified of activity levels of friends, substantially in real-time. One benefit of this feature relates to insurance companies. By using a friend reporter system, users' physical activity level can increase significantly (e.g., 50%) and users can enjoy significant weight loss.

In one or more implementations, groups of users can be established, and groups having similar interests or backgrounds can use the teachings herein to team up and share information with group users. For example, there can be two initial group types: Organizations (e.g., corporate groups), Teams (e.g., user groups), Teams can be created by all users and they are open by default. Group challenges can be created, and the newsfeed can be extended with news items from group users who can be sharing respective elements with their teams or others (e.g., everyone or unlimited). Further, a group directory can be maintained that is searchable and that lists groups that are open or moderated. Users can be prompted to specify their respective locations and fitness interests which can be useful for searching on the group names and descriptions, and suggesting teams to join. This is helpful with getting users socially engaged, and can preclude an empty news feed. In addition, live chat functionality can be supported.

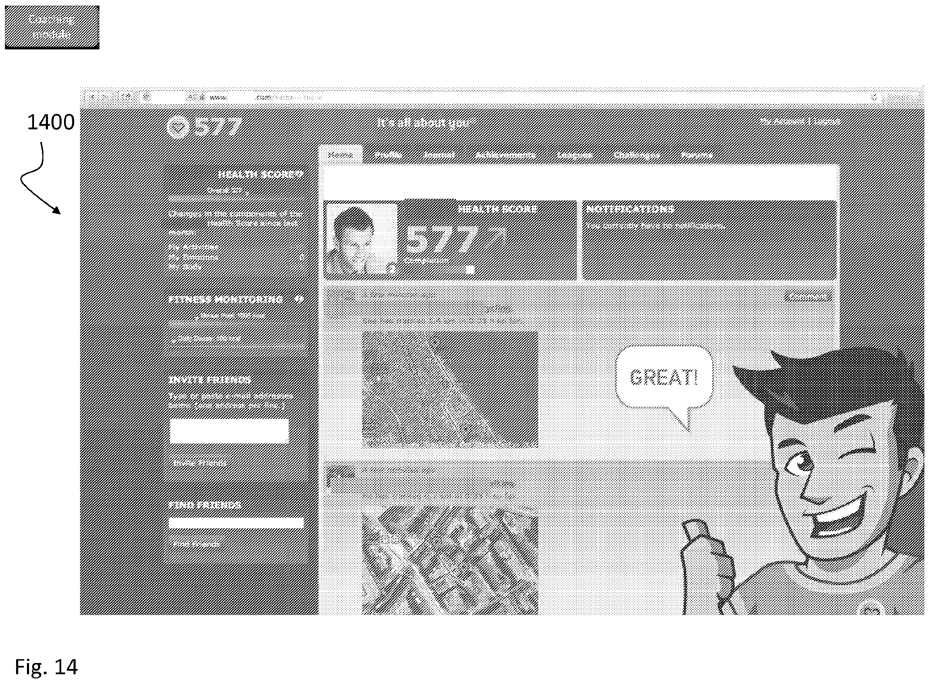

As noted above, in one or more implementations the present application supports use of an avatar and that can be integrated with artificial intelligence. An example of the avatar ("Q") illustrated in the example display screen 1400 in Fig. 14. Multiple behavior levers and novel techniques can be utilized that are based on research from health psychology, psychotherapy, behavioral economics, and influence supporting participants to opt in to healthier behaviors either on their own or with the assistance of a health coach or avatar: "Q," which can include an intelligent feedback loop, be a personal companion, include light artificial intelligence being used for providing the user with feedback of his lifestyle based on activity, nutrition consumption, stress and sleep. Moreover, the avatar can function as a mascot, be represented by a male or female companion who is there to inspire users to improve their health score and overall life quality, and provide intelligent suggestions based on a user's data input on the system. Further, the avatar can function as a coach for self-defined goals that the user sets, and can further be "brandable" to corporate partners.

Thus, in accordance with one or more implementations, users select a male or female version of the avatar "Q" to be their companion on the system. The avatar "Q" can have two principle roles: to function as a guide and companion when using the system, to let users know about notifications and alerts; and to explain, help and provide a walk-through to users when they first sign tip. A second role of the avatar "Q" is that of coach/trainer. The avatar "Q" can form an integral part of a feedback loop with users - from nudging them to continue working out to setting them concrete training plans the avatar "Q" can be present. The avatar "Q" can appear on both a web platform and in a mobile app and can communicate with users in various ways, including but not limited to speech bubbles. In one or more implementations, the avatar will access content from the various trackers and situations on the platform to allow intelligent interactions with the user. The avatar "Q" can function as a coach to regular users and assist them in their training by providing training plans,

The present application further supports a "physician view," which can invite a user by requesting access in a specific role, such as "Personal Trainer'' or "Physician''. In one or more implementations, the role determines specific access rights for the user. The user can grant or clony access and is made aware of the access rights being granted, e.g., "The health professional WILL be able to READ your WORKOUTS."; "The health professional WILL be able to MODIFY your GOALS." One or more of the following features can be made available to health professional for users that have granted access: A free text comment (specific to the relationship of the health professional to the user); Tags (which can also be used for risk stratification into amber complex, red, etc.); Alerts; Filtering (such as by risk and alert state); Setting of fitness goals (e.g. , Run 5 km for 5 weeks); and Setting of training plans (which can be done manually, or by copying from existing plans).

Furthermore, a recommendation module can be provided in connection with lifestyles. In a Health Professional View, for example, the health professional (physician, nurse, personal trainer, or the like) can provide direct recommendation to a user, in such cases when the user has specifically granted access to the professional. In one or more embodiments, The avatar "Q" provides a corporate customer client, base with innovative lifestyle guidance, which will motivate the user to a more active healthy and happy life. The avatar "Q" can also provide knowledge and activities in a number of areas, such as: Fitness/Sporting activities - if a user has not been active or has only been trying one sport type; Diet/Nutrition - prompting the user to drink enough water over the course of a day (intelligence module would suggest the user drink more water if engaging in a lot of activity that day); Stress .. if a user is registering high stress levels the avatar "Q" will provide him with overview and navigation he can take to his Physician; and Sleep - a user consistently recording poor sleep will be able to review their sleeping patterns and consult for professional advice,

Referring to Fig. 14, the avatar Q is illustrated as a coach that provides a user with the coaching role of the health professional (personal trainer or physician). Thus and in connection with setting goals and training plans, Q can function as a coaching tool.

In one or more implementations, the present application supports an inference engine that, as described in the section above, provides a total integrated lifestyle feedback loop that uses artificial intelligence. The feedback loop engine of the present application can learn and store important statistical lifestyle data of the user that helps the user to navigate through the complexity of life. The feedback loop can look at all aspects of a user's health and begin to establish patterns of their lifestyle. Clients of a corporate customer who experiences stress, unhealthy eating habits or sleep disorders can be able to review these patterns and make necessary changes. Further, the components of the health score Platform are interlinked so as to suggest ways for users to improve their health and their health score, based on an intimate knowledge of the health score. Moreover, data can be preferably kept anonymous and secure in any engine calculation.

In one or more implementations, the present application supports user help, such as in an online or other digital fashion. For example, support can include instruction videos, answers to frequently asked questions (FAQ), contact support, , help with getting started on web platform, and mobile app help screens. For example, help is offered to users via an FAQ online and a support forum function that allows users to report bugs and issues. The tracker application also can also be configured with a dedicated help section.

In accordance with the present application, security is a core feature. In accordance with one or more implementations, communication with devices can be protected by HTTPS using high degree and use of security certificate to protect identity of its servers. User data can be securely protected using current cryptographic methods, and that can break the link between user data and account the data belongs to. A remote data center can be employed with significant logical and physical security, and can employ firewall technology not only on the network layer, but also on the application layer. Accordingly, application data can be sent securely and encrypted to the web platform, and a secure payment system is employed for receiving payments, such as related to subscription fees (e.g., per use, monthly, annual, or the like). Moreover, privacy concerns can be addressed, for example, relating to HIPPA or other regulatory compliance.

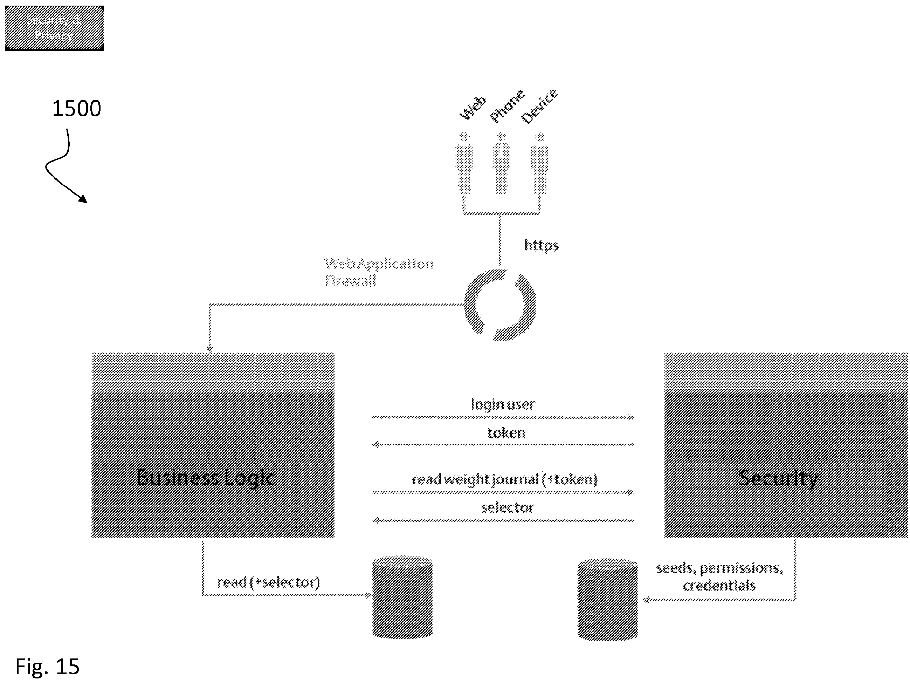

Fig. 15 illustrates an example diagram 1500 illustrating an implementation of the present application that separates a link between health information and account information. After a user logs in, such as by presenting proper credentials, a security server issues a token. In order to access health information (business data), the business logic can request the selector(s) corresponding to that data from the security server, by presenting the token acquired earlier. After the token authorizes the access to the specific data, the security server can provide those selector(s). The business logic then uses the selector(s) to locate the data in the business database. From an architectural point of view, this centralizes security logic in the security server. This is a desirable property, as it makes it easier to maintain the security logic and ensure its correctness (vs. an opposite situation where the security logic is scattered throughout the business logic).

The Health platform 100 according to the present application can be designed as a user centric platform. The user decides in his/her profile settings what kind of information he/she would like to share with friends. In one or more implementations, only a subset of data can be shared with friends on the Health platform 100. Those can include, for example: the user's health score, the user's fitness activities, a profile picture and profile text, achievements, and a list of friends. Other data, such as personal data relating to weight, medical history, lifestyle questions, quality of life questions, blood values, are preferably not accessible or shared on the system.

In an implementations, information is received from a user during a registration process regarding the user's location (e.g., country), email address and password, data points to enable a first health score at first sign-up (e.g., age, gender, weight, height), and acceptance of terms of use. Moreover, a datafoontent repository, content distribution, and blog integration can be provided with social networking sites. in one or more implementations, integration with a content management system ("CMS") of a respective and possibly corporate customer is supported. For example, the health score can be integrated into content specific products of the corporate customer, meaning that the health score can be calculated substantially in real-time and be distributed to alternative client platforms such as the CMS platform of the corporate customer showing the total energy produced or distance of the active users.

In accordance with the present application, relevant parts of the feedback loop logic reside in the individual subsystems of the platform. Accordingly, a rule engine is implementation for notifications that include programming logic that reside in the various subsystems, such as a Forums system, a News system; and/or a Workout system. These rule engines can submit notifications to the feedback loop system. The feedback loop system itself can be construed in terms of a notification scheduler that runs processors on queued notifications in order to eventually deliver those notifications over channels to users. Moreover, a notification domain can be assigned to each notification, which allows users to choose delivery channels per notification domain. This simplifies the user experience.

In one or more implementations, health score information can be provided in an integrated fashion with, for example, one or more of social networking, location information, achievements of friends, nutrition tracker, an inbox, avatar(s), challenges, and invitation.

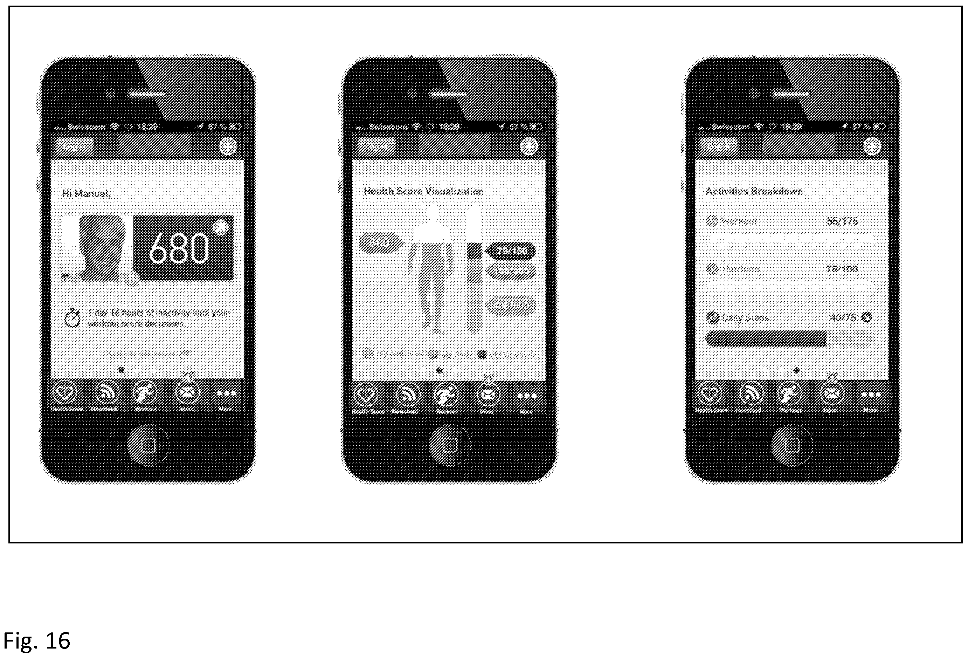

Fig. 16 illustrates mobile computing devices running a mobile application, in accordance with implementations of the present application. For example, a health score is displayed, which includes a rising arrow to represent improvement, and a timer function associated with a workout score is provided. Further, a health score visualization is provided that displays relative values in connection with the user's activities, the user's body and the user's emotions. Moreover, an activities breakdown is displayed in connection with workouts, nutrition and daily stops,

Thus, as shown and described herein, the present application provides for information to be received from users and devices, and processed to provide alerts and notifications. In one or more implementations, one or more rule engines can be provided that periodically and/or continuously generate notifications to users. Particular implementations can depend on a respective subsystem and its specific notification requirements. The notifications can be core information elements driving the feedback loop. Generally, the notifications can be characterized as follows: notifications can be feedbacks or questionnaires; notifications can be presented by an interactive avatar. Moreover, the notification generating rule engines can be part of a individual subsystems generating those notifications. The feedback loop system can provide a generic infrastructure for scheduling, processing, and delivering notifications over various channels.

Regardless of the implementation, the system provides a means for assigning a numerical value that represents the relative health of an individual. The numerical value is described herein as a "health score" and can be used to assess to the individual's health based on health related information collected from a user. The health score is calculated based on the collected health infonnation using an algorithm. The user or the communication subsystem provides the system the health related information concerning a number of health parameters. Predetermined weighting factors are used to assign a relative value of each of the parameters that are used to calculate the health score. The user's health score is then calculated by combining the weighted parameters in accordance with an algorithm. For example, the parameters can be a person's blood glucose level and body weight. A weighting factor "a" is applied to the blood glucose data and a weight, factor "b" can be applied to the body weight data. If the blood glucose data is a more important factor in determining a person's health than body weight, then the weighting factor "a" will be larger than weighting factor "b" so that the blood glucose data has a larger impact on the calculated health score (e.g., Flealthscore = Glucose'a + (Weight/100)*b). In certain implementations, the weighting factor is a non-unity value (e.g., greater or less than one, but not one). Fewer or additional factors can be included in the calculation of the health score, and an offset value can be included that is added or subtracted or which modifies the entire calculation, in certain implementations such as to account for age or gender as two possible reasons, however, the foregoing is intended as a non-limiting example of how to calculate a health score. Other parameters that can be measured and included in the calculation include blood pressure measurements, height, body mass index, fat mass, medical conditions such as diabetes, ventricular hypertrophy, hypertension, irregular heartbeat and fasting glucose values. Where absent, a parameter can be omitted from the calculation or it can be estimated from other parameters and/or values obtained from a sample group of individuals having similar parameters.

In addition to intrinsic medical parameters, physical activity of a user is also taken into account when calculating his or her health score. As noted herein, physical activity can be monitored by the health band 101 via an appropriate sensor dependent on the activity. Sensors can include a GPS unit, an altimeter, a depth meter, a pedometer, a cadence sensor, a velocity sensor, a heart rate monitor or the like. In the case of gym-based activities, computerized exercise equipment can be configured to provide data directly on the program completed by the user (for example, a so-called elliptical/cross-trainer can provide far better data on the workout than a user's pedometer etc). Although automated capture of parameters concerning a user's physical activity is preferred, a user interface for manual activity entry can be also provided. In this regard, an exercise machine such as a treadmill, elliptical, stationary bike or weight lifting machine with a rack of weights or bands can be provided with a communications interface to communicate with the system described herein to provide extrinsic physical activity parameters to the system and to receive and further include a processor configured to process data from the system so as to automatically adjust an exercise program at the exercise machine to meet a goal, challenge, or other objective for that user.

Lifestyle data such as diet, smoking, alcohol consumed and the like can also be collected and used in calculating the health score. In one embodiment, a barcode or RFID scanner can be used by a user to capture data on food and/or nutrition that is consumed, and that can be then translated at a remote system, such as the server or a website in communication with the server, into parameters such as daily calorie, fat and salt intake. In part, the system relies on such data being provided by the user while other data can be obtained through data network connections once permissions and connectivity rights are in place.

Physical activity and lifestyle data can be tracked over time and a decay algorithm can be applied when calculating its effect on the health score, as is discussed in more detail below. As such, physical activity far in the past has a reduced positive effect on the health score. Preferably, the weighting factors used in the algorithm for the computation of the health score are adjusted over time in accordance with a decay component which can be arranged to reduce the relative weight of the parameters that are used in the calculation. The decay component can itself comprise a weighting value, but can also comprise an equation that takes into account at least one factor associated specifically with the user, such as the user's weight or weight range, age or age range, any medical conditions known to the system, and any of the other parameters that may be known to the system, or a curve that can be configured in view of these factors so that a value can be read from the curve as a function of the values along the axes for that user. In this way, the decay component can reduce the relative weight of the parameters used in the health score calculation for a first user differently than for another user, such as when the first user has a first age or age range and the second user has a second age or age range.

A central system, preferably a database and website that can be hosted, for example, by the information processor 102, maintains data on each user and his or her health score and associated parameters and their trends over time. The data can be maintained in such a way that sensitive data can be stored independent of human identities, as understood in the art.

The calculated health score for each user can be then processed in dependence on a system, group or user profile at the central system. Depending on the profile settings, the health score and trends associated can cause various automated actions. For example, it can cause: triggering of an automated alert; providing user feedback such as a daily email update; triggering the communication of automated motivation, warnings and/or goal setting selected to alleviate a perceived issue; adjustment of a training program; or automated referral for medical analysis.

The user's health score can be also provided to a designated group of recipients via a communication portal. The group of recipients can comprise selected, other, users of the system (e.g., friends and family) so that the health scores of the selected, other users can be compared against the health score of still others. In alternative arrangements, all users can see other user's scores, or the group of recipients can be defined as a specific health insurance provider so that price quotes can be provided to insure the individual. Other possibilities are within the scope the invention.

A data collection module executing on the processor can prompt a user to provide health related data corresponding to a number of parameters, in one implementation, one or more the parameters are provided automatically by the communication subsystem. The parameters can include the user's body weight, height, and age and fitness activity information. Such measurable medical parameters are intrinsic parameters of the user. The user's body weight and height provide information about the user's current state of health. The fitness activity information corresponds to the amount of exercise the user engages in. This information is an example of a physically activity parameter that is an extrinsic parameter of the user. For example, the user can enter information about his or her daily fitness activities, such as the amount of time the user engaged in physical activity and the type of physical activity, if the user went to the gym and exercised on a bicycle for thirty minutes, for example, that information can be entered into the system. The user's fitness activity information provides information about the actions that are being taken by the user in order to improve his or her fitness.

A user's body weight, height, age and fitness activity information are just some of the parameters for which information can be collected. The system can collect, and process a multitude of other parameters that can be indicative of a user's health. For example, parameters can irichide blood glucose levels, blood pressure, blood chemistry data (e.g., hormone levels, essential vitamin and mineral levels, etc.), cholesterol levels, immunization data, pulse, blood oxygen content, information concerning food consumed (e.g., caloric, fat, fiber, sodium content), body temperature, which are just some of a few possible, non-limiting examples of parameters that can be collected. Various other parameters that are indicative of a person's health that can be reliably measured could be used to calculate a person's health score.

A weighting module can recall weighting factors from the memory. The weighting factors can be multiplication coefficients that are used to increase or decrease the relative value of each health parameters. A weighting factor can be assigned to each health parameter as shown in the formulas herein. The weighting factors are used to control the relative values of the health parameters. Some health parameters are more important than others in the calculation of the users' health score. Accordingly, weighting factors are applied to the health parameters increase or decrease the relative affect each factor has in the calculation of the user's health score. For example, a user's current body weight can be more important than the amount of fitness activity the user engages in. In this example, the body weight parameter would be weighted more heavily by assigning a larger weighting factor to this parameter. The weighting module applies the recalled weighting factors to the collected health parameter values to provide weighted health parameter values. The weighting factor can be zero in which case a particular parameter has no impact on the health score. The weighting factor can be a negative value for use in some algorithms.

After the parameters have been weighted, the user's health score can be computed via a scoring module operating in the processor. The scoring module combines the weighted parameters according to an algorithm. In one implementation, the health score can be the average of the user's body mass index (BMI) health score and the user's fitness health score minus two times the number of years a person is younger than 95. The algorithm formula for this example is reproduced below;

$$\text{health score} = ((\text{BMI Healthscore} + \text{Fitness Healthscore})/2) - 2*(95\text{-Age}).$$

The user's BMI Healthscore can be a value between 0 and 1000. The BMI Healthscore is based on the user's BMI, which is calculated based on the user's weight and height, and how much the user's BMI deviates from what is considered a healthy BMI. A chart or formula can be used to normalize the user's BMI information so that dissimilar information can be combined. A target BMI value is selected which is assigned a maximum point value (e.g. 1000). The more the user's BMI deviates from the target value the fewer points are awarded. The user's Fitness Heahhscore is based on the physical activity or exercise of a person. In one embodiment, it is the sum of the number of fitness hours (i.e., the amount of time the user engaged in fitness activities) in the past 365 days where each hour is linearly aged over that time so that less recent activity is valued less. The resulting sum can be multiplied by two and tapped at 1000. This normalized the fitness information so that it can be combined to arrive at the health score. A target daily average of fitness activity is selected and is awarded the maximum amount of points (e.g. 1000). The user is awarded fewer points based on how much less exercise that is engaged in compared to the target.

in another implementation, the health score is determined from a number of sub-scores that are maintained in parallel beyond the BMI health score and the fitness health score. Likewise, the health score can be determined using similar information in a combinative algorithm as discussed above using different or no age adjustments.

Intrinsic medical parameters are processed to determine a base health score. Extrinsic parameters such as those from physical exercise are processed to determine a value that is allocated to a health pool and a bonus pool. The value, preferably expressed in MET hours, associated with a physical activity is added to both the health pool and the bonus pool. A daily decay factor is applied to the bonus pool. Any excess decay that cannot be accommodated by the bonus pool is then deducted from the health pool. The amount of decay is determined dependent on the size of the health pool and bonus pool such that a greater effort is required to maintain a high health and bonus pool. The health pool value is processed in combination with the score from the intrinsic medical parameters in order to calculate the overall health score value. In one embodiment, the health pool value is a logarithm or other statistical function is applied to age the respective values over time such that only the most recent activity is counted as being fully effective to the health/bonas pool.

The health score can be based on a weighted combination of health factor(s) and the exercise record of the person over time. The health factors can be updated regularly by the user. For example, the user can provide health related information after every event that is tracked and processed by the system. The user can update after a meal, after exercising, after weighing himself, etc. In the case of recordal of an activity/event by a sensor, portable device or the like, the captured/calculated parameters can be automatically uploaded and used to produce a revised health score. For example, feedback could be provided showing the effect of exercise while a user is running, working out on exercise equipment etc. In selected embodiments, feedback can be provided to an administrator such as a gym staff member where it is determined that a user is exceeding a predetermined threshold (which due to knowledge of their health can be varied respective to their health score or other recorded data). Accordingly, the health related data can be updated in a near real-time manner.

The user can also update the information twice daily, once daily, or at other periodic times. Moreover, the health score can be based on an average of the information over time. Fitness activity, for example, can be averaged over a period of time (e.g. over a week, month, or year). Averaging data over time will reduce the impact to the health score caused by fluctuations in data. Periods in which the data was uncharacteristically high (e.g., the person was engaging large amount of fitness activity over a short period of time) or uncharacteristically low (e.g., person engaged in no fitness activity for a week due to an illness) does not dramatically affect the health score with averaging over time. The health related information can be stored in the memory or in a database accessible by the processor.

The stored data can also be used to predict future health scores for a user. A prediction module can analyze past data (e.g., fitness habits, eating habits, etc.) to extrapolate a predicted health score based on an assumption that the user will continue to act in a predicable manner. For example, if the data shows that a user has exercised one hour every day for the past thirty days, the prediction module can predict, in accordance with a prediction algorithm, that the user will continue to exercise one hour for each of the next three days. Accordingly, the scoring module can calculate a predicted health score at the end of the next three days based on the information from the prediction module. It can also factor the prediction into other actions. For example, the system can suggest a more exerting physical activity level or challenge to someone who has a high health score but is predicted based on past experience to then take a number of days off for recuperation. Furthermore, the system can provide encouragement to the user to maintain a course of activity or modify behavior. For example, the system can send a message to the user indicating that if the user increased fitness activity by a certain amount of time, the health score would go up by a certain amount. This would allow the user set goals to improve health.

The use of the health score allows for a relative comparison of a user's health with that of another person's even though each person can have very different characteristics, which would make a direct comparison difficult. For example, a first user (User 1) can have a very different body composition or engage in very different fitness activities as compared to a second user (User 2), which makes direct comparison of the relative health of each user difficult. The use of the health score makes comparison of the two users possible with relative ease. In one example, User 1 is slightly overweight, which would tend to lower User 1's health score. However, User 1 also engages is large amounts of fitness activities, thereby raising the user's overall health score, in contrast. User 2 has an ideal body weight, which would contribute to a high health score, but engages in very little fitness activity, thereby lowering the health score. User 1 and User 2 are very different in terms of their health related parameters. Accordingly, it would be very difficult to assess and compare the relative health of User 1 and User 2. In accordance with the invention, information related to certain health parameters is collected from User 1 and User 2, which is used to calculate an overall health score. A comparison of User 1's and User 2's health score allows for an easy assessment and comparison of the health of these two users even though they are very different and have very different habits. Therefore, the health score has significant value so that members of a group can compare their relative health and so that other entities (e.g., employers, health care insurers) can assess the health of an individual.

The health parameter data and health scores can be stored over time, in a memory or other database, so that a user can track his or her progress. Charts can be generated in order for a user to track progress and analyze where there can be improvement in behavior. Moreover, trends can be identified that can lead to the diagnosis of medical problems and/or eating habits. For example, if a person's weight is continuing to increase despite the same or increased amount of fitness activity, the system can trigger or suggest that they seek certain medical tests (e.g. a thyroid test, pregnancy test) to determine the cause of the weight gain.

In certain implementations, the majority of the system is hosted remotely from the user and the user accesses the system via a local user interface device. For example the system can be internet based and the user interacts with a local user interface device (e.g., personal computer or mobile electronic device) that is connected to the internet (e.g., via a wire/wireless communication network) in order to communicate data with the internet based system. The user uses the local interface device to access the internet based system in which the memory and software modules are operating remotely and communicating over the internet with the local device. The local device is used to communicate data to the remote processor and memory, in which the data is remotely stored, processed, transformed into a health score, and then provided to the designated groups via a restricted access internet portal. Alternatively, the system can be primarily implemented via a local device in which the data is locally stored, processed, and transformed into a health score, which is then communicated to a data sharing portal for remote publication to the designated groups.

The system can be implemented in the form of a social networking framework that is executed by software modules stored in memory and operating on processors. The system can be implemented as a separate, stand alone "health themed" social networking system or as an application that is integrated with an already existing social networking system (e.g., Facebook, MySpace, etc). The user is provided with a homepage in which the user can enter information, manage which information is published to designated groups, and manage the membership of the designated groups. The homepage includes prompts to the user to enter the health related information for the each of the various parameters. The user can enter his or her weight, date of birth, height, fitness activity, and other health related information. The user's health score is then calculated. The health score is shared with other users that are designated as part of a group permitted to have access to that information. Moreover, the user can view the health score information of others in the group. Accordingly, the user is able to compare his or her overall health with the health of others in the group. Comparison of health scores with others in the group can provide motivation to the individuals in the group to compete to improve their health scores. Other information, such as health tips, medical news, drug information, local fitness events, health services, advertising and discounts for medical and/or fitness related supplies and service, issuance of fitness challenges or health related goals, for example, can be provided via the homepage.

In further implementations, the health score can be a composite of a Metric Health Model score and a Quality of Life Model score. Combining scores from multiple models provides a more holistic assessment of a user's health. The Metric Health Model score assesses a user's health based on relatively easily quantifiable parameters (e.g., age, sex, weight etc.) and compares those numbers to acceptable populations study models. The Quality of Life Model score focus on a user's self-assessed quality of life measure based on responses to a questionnaire (i.e., the system takes into account the user's own assessment of their health and life quality) because there are correlations between how an individual "feels" about his or her life and a realistic measure of health. A combination of the scores from these two models, which will be discussed in more detail below, provides a more inclusive and holistic assessment of health.

The Metric Health Model score can be based on medical parameter information of a user, such as their medical history information, attributes, physiological metrics, and lifestyle information to the system. For example, the system can provide the user a questionnaire to prompt responses (yes/no, multiple choice, numerical input, etc.) or provide the user with form fields to complete. Medical history information can include the user's history of medical conditions and/or the prevalence of medical conditions in the user's family. Examples of medical history information can include information such as whether the user has diabetes, has direct family members with diabetes, whether the user or family members have a history of heart attack, angina, stroke, or Transient Ischemic Attack, a history of atrial fibrillation or irregular heartbeat, whether the user or family members have high blood pressure requiring treatment, whether the user or family members have hypothyroidism, rheumatoid arthritis, chronic kidney disease, liver failure, left ventricular hypertrophy, congestive heart failure, regular use of steroid tablets, etc.

In one more implementations, the Metric Health Model score can also be based on user attributes. The attributes can include age, sex, ethnicity, height, weight, waist size, etc. In addition, Metric Health Model score can be based on physiological metrics of the user. Examples of physiological metrics can include systolic blood pressure, total serum cholesterol, high-density lipoprotein (HDL), low-density lipoprotein (LDL), triglycerides, high-sensitivity C-reactive protein, fasting blood glucose, etc. The inputs can also include parameters of a user's lifestyle. For example, lifestyle parameters can include inputs about whether the user is a smoker (ever smoked, currently smokes, level of smoking, etc.), how much exercise the user performs (frequency, intensity, type, etc.), type of diet (vegetarian, high-protein diet, low-fat diet, high-fiber diet, fast-food, restaurant, home cooking, processed and pre-packaged foods, size of meals, frequency of meals, etc.). These are some of the examples of parameters that can be used to compare the user's health indicators to survival probability models in order to calculate the user's Metric Health Model score.

The Metric Health Model score can be calculated by comparing the user's medical parameter information to survival probability models. A score, preferably in the range of 0 to 1000, with the top end signifying perfect health and the low side signifying poor health, can be derived following a two-step process. First, an overall survival probability is obtained from a combination of the survival probabilities generated by individual survival probability models, as described above. Second, the resulting survival probability, which is a number in the 0 to 1 range, is transformed using a parametric nonlinear mapping function into the 0 to 1000 range. The parametric mapping function is tuned so that it is linear, with a high slope, in the region of typical survival probabilities, and asymptotically slopes off in the low and high ends of the survival probability distribution. The mapping function is designed to be strongly reactive to changes in the typical survival probability region.

As discussed above, the health score can be composed of the Metric Health Model score, and also the Quality of Life Model score. The Quality of Life Model score is based on a user's answers to a set of questionnaires. The system can include several different questionnaires with some questions in common. The type of questionnaires and the type of questions therein presented to the user can be tailored based on a user's health parameters (i.e., user age, other data in the user's medical history, etc.). A specific questionnaire can be generated and presented to the user on the basis of information on the user that is known to the system. The questions can be presented with an appropriate multiple choice response that the user can check/tick on a form, with no free-form text is entered by the user to permit easier assessment of the responses. Other types of responses are possible (e.g., rating how true a statement is to the user 1-10). The following list provides several sample questions (in no particular order) on a number of health-related quality of life topics that can be used in a system questionnaire.

Thus, in a broad aspect, a method according to the invention can be understood as collecting health related information, processing the information into a health score, and publishing the health score is provided. A system for implementing the method can include a computer having a processor, memory, and code modules executing in the processor for the collection, processing, and publishing of the information, information concerning a plurality of health related parameters of a user is collected, particularly, both intrinsic values concerning the measurable, medical parameters of at least one natural person., and the extrinsic values concerning the activities of each such person(s) such as the exercise performed, the type of job the person has and the amount of physical work associated with the job (e.g. sedentary, desk job versus active, manual labor intensive job) and/or the calories/food consumed. Weighting factors are applied to the health related parameter in order control the relative affect each parameter has on the user's calculated health score. The health score is computed using the processor by combining the weighted parameters in accordance with an algorithm. The health score is published to designated group vi a portal, in one implementation, the portal is an internet based information sharing forum.

This application is a divisional application of EP13849993.4 and claims priority from US61/732203.

**Claims**

1. A computer-implemented method for transmission and notification of information to a computing device, the method comprising:

providing, by at least one processor configured to execute code stored on non-transitory processor readable media, a software application that configures the computing device with:

an interactive user interface to receive from and provide information to a user;
an activity tracking subsystem interface configured to receive information from a subsystem; and
a tracking subsystem interface to receive at least biological and physiological information automatically from at least one of a plurality of respective subsystems that are communicatively coupled to the configured computing device,
wherein the software application further configures the computing device to:
receive, via the user interface, the activity tracking subsystem interface and the tracking subsystem interface, parameters of user health information representing a respective user's i) biological state, ii) physiological state and iii) self-assessed state, and activity information representing the respective user's physical activity;
receiving, by the at least one processor from the configured computing device, the parameters of user health information and the respective activity information;
calculating, by the at least one processor using an algorithm, based on the parameters of the user health information received from the configured computing device and predetermined weighting factors used to assign a relative value of each of the parameters, a first health score representing an assessment of the respective user's health,
**characterized by**:
the first health score comprising a weighted sum of values from applying the parameters to a metric health model, a quality of life model and a lifestyle model, wherein the metric health model includes information from the user about who the user is and comprises a score determined from a probability of the user developing a disease, the quality of life model which includes information from the user about how the user feels and comprises a score determined from a user's answers to a questionnaire, and the lifestyle model which includes information from the user about how the user lives, the value of the lifestyle model for the user comprising a combined score from a plurality of categories to monitor and quantify lifestyle characteristics into a lifestyle score, the categories including fitness, nutrition, stress, background physical activity, weight-management and smoking cessation and being quantified by a score component, a bonus component and a decay function;
quantifying an effect of changing a lifestyle characteristic by the user by determining weights with which the lifestyle characteristic contributes to the lifestyle score from:

the sensitivity of the metric health model score to changes in a set of modifiable risk factors for the user, the set of modifiable risk factors being selected from a set including: weight, body-mass index, waist circumference, total serum cholesterol, high-density lipoprotein, low-density lipoprotein, triglycerides, fasting blood glucose, systolic blood pressure, diastolic blood pressure, C-reactive protein, resting heart rate, heart rate recovery, percent body fat, and smoking status;
a sensitivity matrix that relates the effect of each lifestyle characteristic on each of the modifiable risk factors, wherein the step of quantifying including calculating the combined weight of the modifiable risk factors for each lifestyle category; and
ranking the lifestyle characteristics to order the lifestyle characteristics by potential impact to alter the weighting of the lifestyle score by calculating, by the at least one processor, a difference between the first health score of the user and a second health score value given an ideal, best value of one of the modifiable risk factors, ranking the modifiable risk factors from largest to smallest effect on the lifestyle score, and using the sensitivity matrix to translate the modifiable risk factor ranking to a lifestyle characteristic change ranking;

generating, by the at least one processor an output recommending a priority of lifestyle components to change to the user, based the characteristic change ranking.

2. The method of claim 1, further comprising:

determining, by the at least one processor, at least one of medical diagnostic information, medical benchmark information and medical analytic information; and
providing at least one of the medical diagnostic information, medical benchmark information and medical analytic information, at the configured computing device via the user interface.

3. The method of claim 1, further comprising receiving, via the user interface, medical information associated with at least one of the user's family medical history, the user's demographics and the user's metabolism, and wherein determining the health-related information further includes using the medical information.

4. The method of claim 1, further comprising receiving additional sensed information of the type that is associated at least one of biological information, physiological information and/or physical activity of the respective user;

processing the additional sensed information, by the at least one processor, to provide updated user information;
determining updated health-related information, by the at least one processor, using the updated user information;
transmitting the updated user information and the updated health-related information to the configured computing device; and
providing the updated health-related information at the configured computing device via the user interface.

5. The method of claim 4, further comprising:

comparing, by the at least one processor, at least two of the health-related information, the user information, the updated health-related information and the updated user information;
determining feedback, by the at least one processor, based on the comparison; and
providing the feedback at the configured computing device via the user interface.

6. The method of claim 1, wherein the user interface includes a selectable option for regulating at least one of an amount of health-related information to be displayed, a type of health-related to be displayed, and a frequency of displaying information.

7. A computer-implemented system for transmission and of information to a computing device, the system comprising:

at least one processor configured to execute code stored on non-transitory processor readable media;
a software application that configures the computing device with:

an interactive user interface to receive from and provide information to a user;
an activity tracking subsystem interface configured to receive information from a subsystem; and
a tracking subsystem interface to receive at least biological and physiological information automatically from at least one of a plurality of respective subsystems that are communicatively coupled to the configured computing device,

wherein the software application further configures the computing device to:
receive, via the user interface, the activity tracking subsystem interface and the tracking subsystem interface, parameters of user health information representing a respective user's i) biological state, ii) physiological state and iii) self-assessed state, and activity information representing the respective user's physical activity;
wherein the at least one processor is configured to perform the following steps:

receive, from the configured computing device, the parameters of user health information and the respective activity information;
calculate, using an algorithm, based on the parameters of the user health information received from the configured computing device and predetermined weighting factors used to assign a relative value of each of the parameters, a first health score representing an assessment of the respective user's health,
**characterized by**:

the first health score comprising a weighted sum of values from applying the parameters to a metric health model, a quality of life model and a lifestyle model, wherein the metric health model includes information from the user about who the user is and comprises a score determined from a probability of the user developing a disease, the quality of life model which includes information from the user about how the user feels and comprises a score determined from a user's answers to a questionnaire, and the lifestyle model which includes information from the user about how the user lives, the value of the lifestyle model for the user comprising a combined score from a plurality of categories to monitor and quantify lifestyle characteristics into a lifestyle score, the categories including fitness, nutrition, stress, background physical activity, weight-management and smoking cessation and being quantified by a score component, a bonus component and a decay function;
quantify an effect of changing a lifestyle characteristic by the user by determining weights with which the lifestyle characteristic contributes to the lifestyle score from:

the sensitivity of the metric health model score to changes in a set of modifiable risk factors for the user, the set of modifiable risk factors being selected from a set including: weight, body-mass index, waist circumference, total serum cholesterol, high-density lipoprotein, low-density lipoprotein, triglycerides, fasting blood glucose, systolic blood pressure, diastolic blood pressure, C-reactive protein, resting heart rate,
heart rate recovery, percent body fat, and smoking status;
a sensitivity matrix that relates the effect of each lifestyle characteristic on each of the modifiable risk factors,
wherein the step of quantifying including calculating the combined weight of the modifiable risk factors for each lifestyle category;

rank the lifestyle characteristics to order the lifestyle characteristics by potential impact to alter the weighting of the lifestyle score by calculating a difference between the first health score of the user and a health score value given an ideal, best value of one of the plurality of modifiable risk factors, ranking the modifiable risk factors from largest to smallest effect on the lifestyle score, and using the sensitivity matrix to translate the modifiable risk factor ranking to a lifestyle characteristic change ranking; and,
generate, based on the difference, an output recommending a priority of lifestyle components to change to the user, based the characteristic change ranking.

8. The system of claim 7, wherein the at least one processor is configured to: determine at least one of medical diagnostic information, medical benchmark information and medical analytic information, using the health-related information or the user information; and
provide at least one of the medical diagnostic information, medical benchmark information and medical analytic information, at the configured computing device via the user interface.

9. The system of claim 7, wherein medical information associated with at least one of the user's family medical history, the user's demographics and the user's metabolism is received via the user interface, and
wherein the at least one processor is further configured to determine the health-related information using the medical information.

10. The system of claim 7, wherein the at least one processor is configured to:

receive additional sensed information of the type that is associated at least one of biological information, physiological information and/or physical activity of the user;
process the additional sensed information to provide updated user information;
determine updated health-related information using the updated user information;
transmit the updated user information and the updated health-related information to the configured computing device; and
provide the updated health-related information at the configured computing device via the user interface.

11. The system of claim 10, wherein the at least one processor is configured to:

compare at least two of the health-related information, the user information, the updated health-related information and the updated user information;
determine feedback based on the comparison; and

provide the feedback at the configured computing device via the user interface.

**12.** The system of claim 11, wherein the feedback includes at least one of an alert and a notification.

**13.** The system of claim 7, wherein the comparative information regards at least one of social relations, personal progress, reminders for input and private messaging.

**14.** The system of claim 7, wherein the user interface includes a selectable option for regulating at least one of an amount of health-related information to be displayed, a type of health-related to be displayed, and a frequency of displaying information.

**15.** The system of claim 7, wherein the at least one processor is configured to:

derive several risk factors from lifestyle and metabolic characteristics; and
use the several risk factors as multipliers for a risk model to determine at least one health risk.

**Patentansprüche**

**1.** Computerimplementiertes Verfahren zur Übertragung und Mitteilung von Informationen an eine Computereinrichtung, wobei das Verfahren aufweist:
Bereitstellen einer Softwareanwendung durch mindestens einen Prozessor, der so konfiguriert ist, dass er auf einem nicht-übertragbaren, prozessorlesbaren Medium gespeicherten Code ausführt, die die Computereinrichtung konfiguriert, mit:

einer interaktiven Benutzerschnittstelle, um Informationen von einem Benutzer zu empfangen und ihm bereitzustellen;
einer Schnittstelle eines Subsystems zum Aktivitätstracking, die so konfiguriert ist,
dass sie Informationen von einem Subsystem empfängt; und
einer Schnittstelle für ein Tracking-Subsystem, um zumindest biologische und
physiologische Informationen automatisch von mindestens einem aus einer Mehrzahl von jeweiligen Subsystemen zu empfangen, die kommunikativ mit der konfigurierten Computereinrichtung verbunden sind,
wobei die Softwareanwendung die Computereinrichtung weiter konfiguriert, um:

über die Benutzerschnittstelle, die Schnittstelle des Aktivitätstracking-Subsystems und die Schnittstelle des Tracking-Subsystems, Empfangen von Parametern der Gesundheitsinformationen des Benutzers, die i) den biologischen Zustand, ii) den physiologischen Zustand und iii) den selbst eingeschätzten Zustand des jeweiligen Benutzers darstellen, sowie Aktivitätsinformationen, die die körperliche Aktivität eines jeweiligen Benutzers darstellen;
durch den mindestens einen Prozessor von der konfigurierten Computereinrichtung, Empfangen der Parameter der Gesundheitsinformationen des Nutzers und der jeweiligen Aktivitätsinformationen;
durch den mindestens einen Prozessor unter Verwendung eines Algorithmus, Berechnen, basierend auf den Parametern der von der konfigurierten Computereinrichtung empfangenen Gesundheitsinformationen des Benutzers und
vorbestimmten Gewichtungsfaktoren, die verwendet werden, um jedem der Parameter einen relativen Wert zuzuweisen, eines ersten Gesundheitswerts, der eine Bewertung des Gesundheitszustands des jeweiligen Benutzers darstellt, **gekennzeichnet durch**:

der erste Gesundheitswert weist auf eine gewichtete Summe von Werten aus der Anwendung der Parameter auf ein metrisches Gesundheitsmodell, ein Lebensqualitätsmodell und ein Lebensstilmodell, wobei das metrische Gesundheitsmodell Informationen von dem Benutzer darüber enthält, wer der Benutzer ist, und einen Wert umfasst, der aus einer Wahrscheinlichkeit bestimmt wird, dass der Benutzer eine Krankheit entwickelt, das Lebensqualitätsmodell Informationen von dem Benutzer darüber enthält, wie sich der Benutzer fühlt, und
einen Wert umfasst, der aus Antworten des Benutzers auf einen Fragebogen bestimmt wird, und das Lebensstilmodell Informationen von dem Benutzer darüber enthält, wie der Benutzer lebt, der Wert des Lebensstilmodells für den Benutzer einen kombinierten Wert aus einer Vielzahl von Kategorien umfasst, um Charakteristika des Lebensstils in einen Lebensstilwert zu überwachen und zu quantifizieren, wobei die Kategorien umfassen Fitness, Ernährung, Stress, körperliche Hintergrund-Aktivität, Gewichtsmanagement und Raucherentwöhnung und quantifiziert werden durch eine Wert-Komponente, eine Bonus-Komponente und eine Verfall-Funktion;

Quantifizieren eines Effekts der Änderung einer Lebensstilcharakteristik durch den Benutzer durch Bestimmung von Gewichten, mit denen die Lebensstilcharakteristik zum Lebensstil-Wert beiträgt, von:

der Empfindlichkeit des metrischen Gesundheitsmodell-Werts gegenüber Änderungen in einer Reihe von veränderbaren Risikofaktoren für den Benutzer,

wobei die Reihe der veränderbaren Risikofaktoren aus einer Reihe ausgewählt wird, umfassend: Gewicht, Body-Mass-Index, Taillenumfang, Gesamtcholesterin im Serum, High-Density-Lipoprotein, Low-Density-Lipoprotein, Triglycerid, Nüchternblutzucker, systolischer Blutdruck, diastolischer Blutdruck, C-reaktives Protein, Ruheherzfrequenz, Herzfrequenzerholung, Körperfettanteil und Rauchen-Status;

einer Sensitivitätsmatrix, die die Effekte der einzelnen Lebensstilcharakteristika auf die einzelnen veränderbaren Risikofaktoren in Beziehung setzt,

wobei der Schritt der Quantifizierung das Berechnen des kombinierten Gewichts der veränderbaren Risikofaktoren für jede Lebensstilkategorie enthält; und

das Einordnen der Lebensstilcharakteristika, um die Lebensstilcharakteristika nach potenzieller Auswirkung zu ordnen, um die Gewichtung des Lebensstil-Werts zu ändern, indem durch den mindestens einen Prozessor eine Differenz zwischen dem ersten Gesundheits-Wert des Benutzers und einem zweiten Gesundheits-Wert-Wert berechnet wird, der einen idealen, besten Wert eines der veränderbaren Risikofaktoren ergibt, Einordnen der veränderbaren Risikofaktoren vom größten bis kleinsten Effekt auf den Lebensstil-Wert und Verwenden der Sensitivitätsmatrix, um die Rangfolge der veränderbaren Risikofaktoren in eine Rangfolge der Änderung der Lebensstilcharakteristika umzuwandeln;

Erzeugen, durch den mindestens einen Prozessor, einer Ausgabe, die dem Benutzer eine Priorität der zu ändernden Lebensstilkomponenten empfiehlt, basierend auf der Rangfolge der charakteristischen Änderungen.

2. Verfahren nach Anspruch 1, weiterhin aufweisend:

Durch den mindestens einen Prozessor, Bestimmen von mindestens einer medizinischen Diagnoseinformation, einer medizinischen Benchmark-Information und einer medizinischen Analyseinformation; und

Bereitstellen von mindestens einer der medizinischen Diagnoseinformation, der medizinischen Benchmark-Information und der medizinischen Analyseinformation an die konfigurierte Computereinrichtung über den Benutzerschnittstelle.

3. Verfahren nach Anspruch 1, weiterhin aufweisend, Empfangen, über die Benutzerschnittstelle, von medizinische Informationen, die assoziiert sind mit mindestens einer der medizinische Familienhistorie des Benutzers, demografischen Daten des Benutzers und Stoffwechsel des Benutzers, und

wobei das Bestimmen der gesundheitsbezogenen Informationen ferner die Verwendung der medizinischen Informationen umfasst.

4. Verfahren nach Anspruch 1 weiterhin aufweisend das Empfangen zusätzlicher erfasster Informationen des Typs, die mit mindestens einer der biologischen Information, der physiologischen Information und/oder der körperlichen Aktivität des jeweiligen Benutzers verbunden sind;

Verarbeiten der zusätzlich erfassten Informationen durch den mindestens einen Prozessor, um aktualisierte Benutzerinformationen bereitzustellen;

Bestimmen aktualisierter gesundheitsbezogener Informationen durch den mindestens einen Prozessor unter Verwendung der aktualisierten Benutzerinformationen;

Übermitteln der aktualisierten Benutzerinformationen und der aktualisierten gesundheitsbezogenen Informationen an die konfigurierte Computereinrichtung; und Bereitstellen der aktualisierten gesundheitsbezogenen Informationen auf der konfigurierten Computereinrichtung über die Benutzerschnittstelle.

5. Verfahren nach Anspruch 4, weiterhin aufweisend:

Vergleichen, durch den mindestens einen Prozessor, von mindestens zwei der gesundheitsbezogenen Informationen, der Benutzerinformationen, der aktualisierten gesundheitsbezogenen Informationen und der aktualisierten Benutzerinformationen;

Bestimmen einer Rückmeldung durch den mindestens einen Prozessor auf der Grundlage des Vergleichs; und Bereitstellen der Rückmeldung an der konfigurierten Computereinrichtung über die Benutzerschnittstelle.

6. Verfahren nach Anspruch 1, wobei die Benutzerschnittstelle eine auswählbare Option enthält für das Regulieren mindestens einer aus einer Menge von anzuzeigenden gesundheitsbezogenen Informationen, der Art der anzuzeigenden Gesundheitsinformationen und einer Häufigkeit der Anzeige von Informationen.

7. Computerimplementiertes System zur Übertragung und Mitteilung von Informationen an eine Computereinrichtung, wobei das System aufweist:

mindestens einen Prozessor, der so konfiguriert ist, dass er auf einem nicht-übertragbaren, prozessorlesbaren Medium gespeicherten Code ausführt,

eine Softwareanwendung, die die Computereinrichtung konfiguriert, mit:

einer interaktiven Benutzerschnittstelle, um Informationen von einem Benutzer zu empfangen und ihm bereitzustellen;

einer Schnittstelle eines Subsystems zum Aktivitätstracking, die so konfiguriert ist, dass sie Informationen von einem Subsystem empfängt; und

einer Schnittstelle für ein Tracking-Subsystem, um zumindest biologische und

physiologische Informationen automatisch von mindestens einem aus einer Mehrzahl von jeweiligen Subsystemen zu empfangen, die kommunikativ mit der konfigurierten Computereinrichtung verbunden sind, wobei die Softwareanwendung die Computereinrichtung weiter konfiguriert, um:

über die Benutzerschnittstelle, die Schnittstelle des Aktivitätstracking-Subsystems und die Schnittstelle des Tracking-Subsystems, Parameter der

Gesundheitsinformationen des Benutzers zu empfangen, die i) den biologischen Zustand, ii) den physiologischen Zustand und iii) den selbst eingeschätzten Zustand des jeweiligen Benutzers darstellen, sowie Aktivitätsinformationen, die die körperliche Aktivität eines jeweiligen Benutzers darstellen;

wobei der mindestes eine Prozessor konfiguriert ist, um die folgenden Schritte auszuführen:

Empfangen der Parameter der Gesundheitsinformationen des Nutzers und der jeweiligen Aktivitätsinformationen von der konfigurierten Computereinrichtung;

Berechnen, unter Verwendung eines Algorithmus, basierend auf den Parametern der von der konfigurierten Computereinrichtung empfangenen Gesundheitsinformationen des Benutzers und vorbestimmten Gewichtungsfaktoren, die verwendet werden, um jedem der Parameter einen relativen Wert zuzuweisen, eines ersten Gesundheitswerts, der eine Bewertung des Gesundheitszustands des jeweiligen Benutzers darstellt,

**gekennzeichnet durch**:

der erste Gesundheitswert weist auf eine gewichtete Summe von Werten aus der Anwendung der Parameter auf ein metrisches Gesundheitsmodell, ein Lebensqualitätsmodell und ein Lebensstilmodell, wobei das metrische Gesundheitsmodell Informationen von dem Benutzer darüber enthält, wer der Benutzer ist, und einen Wert umfasst, der aus einer Wahrscheinlichkeit bestimmt wird, dass der Benutzer eine Krankheit entwickelt, das Lebensqualitätsmodell Informationen von dem Benutzer darüber enthält, wie sich der Benutzer fühlt, und

einen Wert umfasst, der aus Antworten des Benutzers auf einen Fragebogen bestimmt wird, und das Lebensstilmodell Informationen von dem Benutzer darüber enthält, wie der Benutzer lebt, der Wert des Lebensstilmodells für den Benutzer einen kombinierten Wert aus einer Vielzahl von Kategorien umfasst, um Charakteristika des Lebensstils in einen Lebensstilwert zu überwachen und zu quantifizieren, wobei die Kategorien umfassen Fitness, Ernährung, Stress, körperliche Hintergrund-Aktivität, Gewichtsmanagement und Raucherentwöhnung und quantifiziert werden durch eine Wert-Komponente, eine Bonus-Komponente und eine Verfall-Funktion;

Quantifizieren eines Effekts der Änderung einer Lebensstilcharakteristik durch den Benutzer durch Bestimmung von Gewichten, mit denen die Lebensstilcharakteristik zum Lebensstil-Wert beiträgt, von:

der Empfindlichkeit des metrischen Gesundheitsmodell-Werts gegenüber Änderungen in einer Reihe von veränderbaren Risikofaktoren für den Benutzer, wobei die Reihe der veränderbaren Risikofaktoren aus einer Reihe ausgewählt wird, umfassend: Gewicht, Body-Mass-Index, Taillenumfang, Gesamtcholesterin im Serum, High-Density-Lipoprotein, Low-Density-Lipoprotein, Triglycerid, Nüchternblutzucker, systolischer Blutdruck, diastolischer Blutdruck, C-reaktives Protein, Ruheherzfrequenz, Herzfrequenzerholung, Körperfettanteil und Rauchen-Status;

einer Sensitivitätsmatrix, die die Effekte der einzelnen Lebensstilcharakteristika auf die einzelnen veränderbaren Risikofaktoren in Beziehung setzt, wobei der Schritt der Quantifizierung das Berechnen des kombinierten Gewichts der veränderbaren Risikofaktoren für jede Lebensstilkategorie enthält;

Einordnen der Lebensstilcharakteristika, um die Lebensstilcharakteristika nach potenzieller Auswirkung zu ordnen, um die Gewichtung des Lebensstil-Werts zu ändern, indem eine Differenz zwischen dem ersten Gesundheits-Wert des Benutzers und einem Gesundheits-Wert-Wert berechnet wird, der einen idealen, besten Wert eines der veränderbaren Risikofaktoren ergibt, Einordnen der veränderbaren Risikofaktoren vom größten bis kleinsten Effekt auf den Lebensstil-Wert und

Verwenden der Sensitivitätsmatrix, um die Rangfolge der veränderbaren Risikofaktoren in eine Rangfolge der Änderung der Lebensstilcharakteristika umzuwandeln;

Erzeugen, basierend auf der Differenz, einer Ausgabe, die dem Benutzer eine Priorität der zu ändernden Lebensstilkomponenten empfiehlt, basierend auf der Rangfolge der charakteristischen Änderungen.

8. System nach Anspruch 7, wobei der mindestens eine Prozessor so konfiguriert ist, um: unter Verwendung der gesundheitsbezogenen Informationen oder der Benutzerinformationen, mindestens eine von medizinischen Diagnoseinformationen, medizinischen Benchmark-Informationen und medizinischen Analyseinformationen zu bestimmen; und mindestens eine der medizinischen Diagnoseinformationen, medizinischen Benchmark-Informationen und medizinischen Analyseinformationen auf dem konfigurierten Computergerät über die Benutzerschnittstelle bereitzustellen.

9. System nach Anspruch 7, wobei medizinische Informationen, die mit mindestens einer der Familienhistorie des Benutzers, demografischen Daten des Benutzers und Stoffwechsel des Benutzers assoziiert sind, über die Benutzerschnittstelle empfangen werden, und
wobei der mindestens eine Prozessor ferner so konfiguriert ist, dass er die gesundheitsbezogenen Informationen unter Verwendung der medizinischen Informationen bestimmt.

10. System nach Anspruch 1, wobei der wenigstens eine Prozessor konfiguriert ist zum:

Empfangen zusätzlicher erfasster Informationen des Typs, die mit mindestens einer der biologischen Information, der physiologischen Information und/oder der körperlichen Aktivität des jeweiligen Benutzers verbunden sind;
Verarbeiten der zusätzlich erfassten Informationen, um aktualisierte Benutzerinformationen bereitzustellen;
Bestimmen aktualisierter gesundheitsbezogener Informationen unter Verwendung der aktualisierten Benutzerinformationen;
Übermitteln der aktualisierten Benutzerinformationen und der aktualisierten gesundheitsbezogenen Informationen an die konfigurierte Computereinrichtung; und Bereitstellen der aktualisierten gesundheitsbezogenen Informationen auf der konfigurierten Computereinrichtung über die Benutzerschnittstelle.

11. System nach Anspruch 10, wobei der mindestens eine Prozessor konfiguriert ist, zum:

Vergleichen von mindestens zwei der gesundheitsbezogenen Informationen, der Benutzerinformationen, der aktualisierten gesundheitsbezogenen Informationen und der aktualisierten Benutzerinformationen;
Bestimmen einer Rückmeldung auf der Grundlage des Vergleichs; und
Bereitstellen der Rückmeldung auf der konfigurierten Computereinrichtung über die Benutzerschnittstelle.

12. System nach Anspruch 11, wobei die Rückmeldung mindestens eine Warnung oder eine Benachrichtigung enthält.

**13.** System nach Anspruch 7, wobei die vergleichende Information mindestens eines betrachtet aus sozialen Beziehungen, persönlichem Fortschritt, Erinnerungen für Eingaben und private Nachrichten.

**14.** System nach Anspruch 7, wobei die Benutzerschnittstelle eine auswählbare Option enthält für das Regulieren mindestens einer aus einer Menge von anzuzeigenden gesundheitsbezogenen Informationen, der Art der anzuzeigenden Gesundheitsinformationen und einer Häufigkeit der Anzeige von Informationen.

**15.** System nach Anspruch 7, wobei der mindestens eine Prozessor konfiguriert ist, zum:

Ableiten mehrerer Risikofaktoren aus dem Lebensstil und den
Stoffwechselmerkmalen; und
Verwenden der verschiedenen Risikofaktoren als Multiplikatoren für ein Risikomodell, um mindestens einen Gesundheitsrisiko zu bestimmen.

**Revendications**

**1.** - Procédé mis en œuvre par ordinateur pour la transmission et la notification d'informations à un dispositif informatique, le procédé comprenant :

fournir, par au moins un processeur configuré pour exécuter un code stocké sur un support non-transitoire lisible par processeur, une application logicielle qui configure le dispositif informatique avec :

une interface utilisateur interactive pour recevoir en provenance d'un utilisateur et fournir à l'utilisateur des informations ;
une interface de sous-système de suivi d'activité configurée pour recevoir des informations en provenance d'un sous-système ; et
une interface de sous-système de suivi pour recevoir au moins des informations biologiques et physiologiques automatiquement en provenance d'au moins l'un parmi une pluralité de sous-systèmes respectifs qui sont couplés en communication au dispositif informatique configuré,
dans lequel l'application logicielle configure en outre le dispositif informatique pour :
recevoir, par l'intermédiaire de l'interface utilisateur, de l'interface de sous-système de suivi d'activité et de l'interface de sous-système de suivi, des paramètres d'informations de santé d'utilisateur représentant i) l'état biologique, ii) l'état physiologique et iii) l'état autoévalué d'un utilisateur respectif, ainsi que des informations d'activité représentant l'activité physique de l'utilisateur respectif ;
recevoir, par l'au moins un processeur en provenance du dispositif informatique configuré, les paramètres des informations de santé d'utilisateur et les informations d'activité respectives ;
calculer, par l'au moins un processeur à l'aide d'un algorithme, sur la base des paramètres des informations de santé d'utilisateur reçues en provenance du dispositif informatique configuré et de facteurs de pondération prédéterminés utilisés pour attribuer une valeur relative de chacun des paramètres, un premier score de santé représentant une évaluation de la santé de l'utilisateur respectif ;
**caractérisé par** :

le premier score de santé comprenant une somme pondérée de valeurs résultant de l'application des paramètres à un modèle de santé métrique, à un modèle de qualité de vie et à un modèle de mode de vie, dans lequel le modèle de santé métrique comprend des informations provenant de l'utilisateur concernant l'identité de l'utilisateur et comprend un score déterminé à partir d'une probabilité que l'utilisateur développe une maladie, le modèle de qualité de vie comprend des informations provenant de l'utilisateur concernant la façon dont l'utilisateur se sent et comprend un score déterminé à partir des réponses d'un utilisateur à un questionnaire, et le modèle de mode de vie comprend des informations provenant de l'utilisateur concernant la façon dont l'utilisateur vit, la valeur du modèle de mode de vie pour l'utilisateur comprenant un score combiné à partir d'une pluralité de catégories pour surveiller et quantifier des caractéristiques de mode de vie en un score de mode de vie, les catégories comprenant la forme physique, la nutrition, le stress, l'activité physique de fond, la gestion du poids et le sevrage tabagique et étant quantifiées par une composante de score, une composante de bonus et une fonction de déclin ;
quantifier un effet de changement d'une caractéristique de mode de vie par l'utilisateur en déterminant des pondérations avec lesquelles la caractéristique de mode de vie contribue au score de mode de vie parmi :

la sensibilité du score de modèle de santé métrique à des changements d'un ensemble de facteurs de risque modifiables pour l'utilisateur, l'ensemble de facteurs de risque modifiables étant choisi parmi un ensemble comprenant : le poids, l'indice de masse corporelle, le tour de taille, le cholestérol sérique total, les lipoprotéines de haute densité, les lipoprotéines de basse densité, les triglycérides, la glycémie à jeun, la pression artérielle systolique, la pression artérielle diastolique, la protéine C-réactive, la fréquence cardiaque au repos, la récupération de la fréquence cardiaque, le pourcentage de graisse corporelle et le statut tabagique ;

une matrice de sensibilité qui associe l'effet de chaque caractéristique de mode de vie à chacun des facteurs de risque modifiables ;

dans lequel l'étape de quantification comprend calculer la pondération combinée des facteurs de risque modifiables pour chaque catégorie de mode de vie ; et

classer les caractéristiques de mode de vie afin d'ordonner les caractéristiques de mode de vie par impact potentiel pour modifier la pondération du score de mode de vie en calculant, par l'au moins un processeur, une différence entre le premier score de santé de l'utilisateur et une deuxième valeur de score de santé correspondant à une meilleure valeur idéale de l'un des facteurs de risque modifiables, en classant les facteurs de risque modifiables du plus grand au plus petit effet sur le score de mode de vie, et en utilisant la matrice de sensibilité pour traduire le classement des facteurs de risque modifiables en un classement de changement de caractéristiques de mode de vie ;

générer, par l'au moins un processeur, un résultat recommandant à l'utilisateur une priorité des composantes de mode de vie à changer, sur la base du classement de changement de caractéristiques.

2. - Procédé selon la revendication 1, comprenant en outre :

déterminer, par l'au moins un processeur, au moins l'un parmi des informations de diagnostic médical, des informations de référence médicale et des informations d'analyse médicale ; et

fournir au moins l'un parmi les informations de diagnostic médical, les informations de référence médicale et les informations d'analyse médicale, au dispositif informatique configuré par l'intermédiaire de l'interface utilisateur.

3. - Procédé selon la revendication 1, comprenant en outre la réception, par l'intermédiaire de l'interface utilisateur, d'informations médicales associées à au moins l'un parmi des antécédents médicaux familiaux de l'utilisateur, des données démographiques de l'utilisateur et le métabolisme de l'utilisateur, et

dans lequel la détermination des informations relatives à la santé comprend en outre l'utilisation des informations médicales.

4. - Procédé selon la revendication 1, comprenant en outre recevoir des informations détectées supplémentaires du type de celles qui sont associées à au moins l'un parmi des informations biologiques, des informations physiologiques et/ou l'activité physique de l'utilisateur respectif ;

traiter les informations détectées supplémentaires, par l'au moins un processeur, pour fournir des informations d'utilisateur mises à jour ;

déterminer les informations relatives à la santé mises à jour, par l'au moins un processeur, à l'aide des informations d'utilisateur mises à jour ;

transmettre les informations d'utilisateur mises à jour et les informations relatives à la santé mises à jour au dispositif informatique configuré ; et

fournir les informations relatives à la santé mises à jour au dispositif informatique configuré par l'intermédiaire de l'interface utilisateur.

5. - Procédé selon la revendication 4, comprenant en outre :

comparer, par l'au moins un processeur, au moins deux parmi les informations relatives à la santé, les informations d'utilisateur, les informations relatives à la santé mises à jour et les informations d'utilisateur mises à jour ;

déterminer une rétroaction, par l'au moins un processeur, sur la base de la comparaison ; et

fournir la rétroaction au dispositif informatique configuré par l'intermédiaire de l'interface utilisateur.

6. - Procédé selon la revendication 1, dans lequel l'interface utilisateur comprend une option sélectionnable pour réguler au moins l'un parmi une quantité d'informations relatives à la santé à afficher, un type d'informations relatives à la santé à afficher et une fréquence d'affichage d'informations.

7. - Système mis en œuvre par ordinateur pour la transmission d'informations à un dispositif informatique, le système comprenant :

au moins un processeur configuré pour exécuter un code stocké sur un support non-transitoire lisible par processeur ;
une application logicielle qui configure le dispositif informatique avec :

une interface utilisateur interactive pour recevoir en provenance d'un utilisateur et fournir à l'utilisateur des informations ;
une interface de sous-système de suivi d'activité configurée pour recevoir des informations en provenant d'un sous-système ; et
une interface de sous-système de suivi pour recevoir au moins des informations biologiques et physiologiques automatiquement en provenance d'au moins l'un parmi une pluralité de sous-systèmes respectifs qui sont couplés en communication au dispositif informatique configuré,

dans lequel l'application logicielle configure en outre le dispositif informatique pour :
recevoir, par l'intermédiaire de l'interface utilisateur, de l'interface de sous-système de suivi d'activité et de l'interface de sous-système de suivi, des paramètres d'informations de santé d'utilisateur représentant i) l'état biologique, ii) l'état physiologique et iii) l'état autoévalué d'un utilisateur respectif, et des informations d'activité représentant l'activité physique de l'utilisateur respectif ;
dans lequel l'au moins un processeur est configuré pour effectuer les étapes suivantes :

recevoir, en provenance du dispositif informatique configuré, les paramètres des informations de santé d'utilisateur et les informations d'activité respectives ;
calculer, à l'aide d'un algorithme, sur la base des paramètres des informations de santé d'utilisateur reçues en provenance du dispositif informatique configuré et de facteurs de pondération prédéterminés utilisés pour attribuer une valeur relative de chacun des paramètres, un premier score de santé représentant une évaluation de la santé de l'utilisateur respectif ;

**caractérisé par** :

le premier score de santé comprenant une somme pondérée de valeurs résultant de l'application des paramètres à un modèle de santé métrique, à un modèle de qualité de vie et à un modèle de mode de vie, dans lequel le modèle de santé métrique comprend des informations provenant de l'utilisateur concernant l'identité de l'utilisateur et comprend un score déterminé à partir d'une probabilité que l'utilisateur développe une maladie, le modèle de qualité de vie comprend des informations provenant de l'utilisateur sur la façon dont l'utilisateur se sent et comprend un score déterminé à partir des réponses d'un utilisateur à un questionnaire, et le modèle de mode de vie comprend des informations provenant de l'utilisateur concernant la façon dont l'utilisateur vit, la valeur du modèle de mode de vie pour l'utilisateur comprenant un score combiné à partir d'une pluralité de catégories pour surveiller et quantifier des caractéristiques de mode de vie en un score de mode de vie, les catégories comprenant la forme physique, la nutrition, le stress, l'activité physique de fond, la gestion du poids et le sevrage tabagique et étant quantifiées par une composante de score, une composante de bonus et une fonction de déclin ;
quantifier un effet de changement d'une caractéristique de mode de vie par l'utilisateur en déterminant des pondérations avec lesquelles la caractéristique de mode de vie contribue au score de mode de vie à partir de :

la sensibilité du score de modèle de santé métrique à des changements d'un ensemble de facteurs de risque modifiables pour l'utilisateur, l'ensemble de facteurs de risque modifiables étant choisi parmi un ensemble comprenant : le poids, l'indice de masse corporelle, le tour de taille, le cholestérol sérique total, les lipoprotéines de haute densité, les lipoprotéines de basse densité, les triglycérides, la glycémie à jeun, la pression artérielle systolique, la pression artérielle diastolique, la protéine C-réactive, la fréquence cardiaque au repos, la récupération de la fréquence cardiaque, le pourcentage de graisse corporelle et le statut tabagique ;
une matrice de sensibilité qui associe l'effet de chaque caractéristique de mode de vie à chacun des facteurs de risque modifiables ;
dans lequel l'étape de quantification comprend calculer la pondération combinée des facteurs de risque modifiables pour chaque catégorie de mode de vie ;

classer les caractéristiques de mode de vie afin d'ordonner les caractéristiques de mode de vie par impact potentiel pour modifier la pondération du score de mode de vie en calculant une différence entre le premier score de santé de l'utilisateur et une valeur de score de santé correspondant à une meilleure valeur idéale de l'un parmi la pluralité de facteurs de risque modifiables, en classant les facteurs de risque modifiables du plus grand au plus petit effet sur le score de mode de vie, et en utilisant la matrice de sensibilité pour traduire le classement de facteurs de risque modifiables en un classement de changement de caractéristiques de mode de vie ; et

générer, sur la base de la différence, un résultat recommandant à l'utilisateur une priorité des composantes de mode de vie à changer, sur la base du classement de changement de caractéristiques.

8. - Système selon la revendication 7, dans lequel l'au moins un processeur est configuré pour :

déterminer au moins l'un parmi des informations de diagnostic médical, des informations de référence médicale et des informations d'analyse médicale, à l'aide des informations relatives à la santé ou des informations d'utilisateur ; et

fournir au moins l'un parmi les informations de diagnostic médical, les informations de référence médicale et les informations d'analyse médicale au dispositif informatique configuré par l'intermédiaire de l'interface utilisateur.

9. - Système selon la revendication 7, dans lequel des informations médicales associées à au moins l'un parmi les antécédents médicaux familiaux de l'utilisateur, les données démographiques de l'utilisateur et le métabolisme de l'utilisateur sont reçues par l'intermédiaire de l'interface utilisateur, et

dans lequel l'au moins un processeur est en outre configuré pour déterminer les informations relatives à la santé à l'aide des informations médicales.

10. - Système selon la revendication 7, dans lequel l'au moins un processeur est configuré pour :

recevoir des informations détectées supplémentaires du type de celles qui sont associées à au moins l'un parmi les informations biologiques, les informations physiologiques et/ou l'activité physique de l'utilisateur ;

traiter les informations détectées supplémentaires pour fournir des informations d'utilisateur mises à jour ;

déterminer des informations relatives à la santé mises à jour à l'aide des informations d'utilisateur mises à jour ;

transmettre les informations d'utilisateur mises à jour et les informations relatives à la santé mises à jour au dispositif informatique configuré ; et

fournir les informations relatives à la santé mises à jour au dispositif informatique configuré par l'intermédiaire de l'interface utilisateur.

11. - Système selon la revendication 10, dans lequel l'au moins un processeur est configuré pour :

comparer au moins deux parmi les informations relatives à la santé, les informations d'utilisateur, les informations relatives à la santé mises à jour et les informations d'utilisateur mises à jour ;

déterminer une rétroaction sur la base de la comparaison ; et

fournir la rétroaction au dispositif informatique configuré par l'intermédiaire de l'interface utilisateur.

12. - Système selon la revendication 11, dans lequel la rétroaction comprend au moins l'une parmi une alerte et une notification.

13. - Système selon la revendication 7, dans lequel les informations comparatives concernent au moins l'un parmi des relations sociales, des progrès personnels, des rappels de saisie et une messagerie privée.

14. - Système selon la revendication 7, dans lequel l'interface utilisateur comprend une option sélectionnable pour réguler au moins l'un parmi une quantité d'informations relatives à la santé à afficher, un type d'informations relatives à la santé à afficher, et une fréquence d'affichage d'informations.

15. - Système selon la revendication 7, dans lequel l'au moins un processeur est configuré pour :

déduire plusieurs facteurs de risque à partir du mode de vie et de caractéristiques métaboliques ; et

utiliser les différents facteurs de risque comme multiplicateurs pour un modèle de risque afin de déterminer au moins un risque pour la santé.

Fig. 1

Fig. 2

Fig. 2

EP 3 680 913 B1

Fig. 3A

Fig. 3B

Model of the Nutrition Tracker

**400**

**Triage Questions (User Profiling)**
(e.g. How much are you interested in nutrition?)

*answers*

**Chronology 1 Questions**
High-level questions
(e.g. What do you usually drink with meals?)

*Answers, hints, scores*

User selects a goal

**Chronology 2 Questions**
Specific questions
(e.g. Did you drink water today?)

*Answers, hints, scores*

At the earliest 3 months after the goal was selected

*Answers, hints, scores*

**Chronology 3 Questions**
Specific questions to check the achievement of the selected goal
(e.g. Look back at the past month: On how many days a week did you drink water?)

Fig. 4A

EP 3 680 913 B1

Fig. 4B

EP 3 680 913 B1

**404**

Swisscom 📶 4:38 PM

🔒 Manual Entry

m._____.com/journal/v C    Yahoo!

🏠 **Home**    **Manual Entry**

Please use this form to insert a manual entry into your journal. Workouts can be entered for up to 30 days of history.

⊖ **Activity**

Please choose an activity from the list below.

Activity

**Swimming (Front Crawl)** ⊙

◀    ⤴    📖    2

Fig. 4C

406

Fig. 4D

500

Fig. 5

EP 3 680 913 B1

Fig. 6

Fig. 7

800

Fig. 8

Fig. 9

Goal Setting

1000

| Activities | Places | Calendar | Custom | ← Previous

Duration  ___  Energy  ___  Normalized Energy  ___  Ascent  ___
Distance  ___  Power  403 W  Normalized Power  ___  Descent  ___
Speed  6.1 km/h

Fig. 10A

Fig. 10B

Goal Setting

1004

Achievement in Progress                                    ☐ Goal

Burned: 1'000 MET h                                        **10**

Transfer 1'000 MET h through fitness activities.

By when do you want to reach this goal?

◀                                                    ▶

S  M  T  W  T  F  S

Set Goal

EP 3 680 913 B1

Fig. 10C

Fig. 10D

1006

Fig. 11A

Fig. 11B

Fig. 12

EP 3 680 913 B1

Fig. 13

1400

Fig. 14

EP 3 680 913 B1

Fig. 15

Fig. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61732203
- WO 2012050969 A1
- EP 13849993
- US 61732203 B